# EUROPÄISCHE PATENTSCHRIFT

(11) **EP 4 017 344 B1**
(45) Veröffentlichungstag und Bekanntmachung des Hinweises auf die Patenterteilung: **09.10.2024**
(21) Anmeldenummer: 20767466.4
(22) Anmeldetag: 19.08.2020
(51) Int. Cl.: A61B 5/00, G16H 50/20, A61B 5/01, A61B 5/145, A61B 5/021, A61B 5/024

(54) **VORRICHTUNG ZUR EINWIRKUNG AUF DAS KÖRPERGEWEBE, VERFAHREN ZUR MESSUNG UND AUSWERTUNG EINES KÖRPERGEWEBEZUSTANDES**
APPARATUS FOR INFLUENCING BODY TISSUE, METHOD FOR MEASURING AND EVALUATING A BODY TISSUE CONDITION
APPAREIL POUR INFLUENCER UN TISSU CORPOREL, PROCÉDÉ POUR MESURER ET ÉVALUER UN ÉTAT DE TISSU CORPOREL

(30) Priorität: 20.08.2019 DE 102019005789
(43) Veröffentlichungstag der Anmeldung: 29.06.2022
(73) Patentinhaber: Boeger, David, 8590 Romanshorn (CH)
(72) Erfinder: Boeger, David, 8590 Romanshorn (CH)
(74) Vertreter: Jendricke, Susann
(86) Internationale Anmeldenummer: PCT/EP2020/025380
(87) Internationale Veröffentlichungsnummer: WO 2021/032320

(56) Entgegenhaltungen:
- WO-A2-2012/106593
- US-A1- 2011 082 384
- US-A1- 2019 117 119

## Beschreibung

Die Erfindung betrifft eine Vorrichtung zur Einwirkung auf das Körpergewebe eines Lebewesens.

Das Einwirken auf Körpergewebe kann unterschiedliche Zielrichtungen verfolgen. Bspw. können bezüglich gesundem Körpergewebe Untersuchungen angestellt werden, die die Toleranz gegenüber Kraftbeaufschlagungen verschiedener Art betreffen. Solche Untersuchungen am gesunden Körpergewebe können wichtig für die Kraftbeaufschlagung von beschädigtem Körpergewebe oder Narbengewebe sein. Außerdem kann die Zielrichtung verfolgt werden, Schmerzen im Körpergewebe zu beseitigen oder auch schmerzvorbeugend auf das Körpergewebe einzuwirken.

Beim Körpergewebe kann es sich um Haut, Gelenke, Muskulatur, Bänder und Sehnen, Bindegewebe oder Narbengewebe handeln. Das Bindegewebe umgibt alle Zellstrukturen des Körpers, wie Organe, Muskeln. Dort kann sich auch Narbengewebe befinden, das von außen nicht sichtbar ist. Schmerzen entstehen durch eine pathologische Veränderung des Körpergewebes. Eine pathologische Veränderung kann beispielsweise durch Dauerbelastung, insbesondere bei der Berufsausübung von Berufsfahrern, Schneidern, Friseuren, Büroangestellten, oder beispielsweise durch Gewalteinwirkung entstehen. Das Körpergewebe könnte dahingehend pathologisch verändert sein, dass Faszienverklebungen oder Faszienaufblähungen infolge von Stauungen entstehen und so die Funktionstüchtigkeit der Faszien eingeschränkt wird. Körpergewebeveränderungen wirken sich auch auf andere Körperteile aus, die in denselben Faszienketten- oder -netzen liegen, wie die betroffene Körperregion. Bei dem speziellen Gebiet des Narbengewebes spielt die Beeinträchtigung der Flüssigkeitszirkulation und der Faszienverbindungen im Körper eine besondere Rolle. Derartige Beeinträchtigungen der Faszienverbindungen und der Flüssigkeitszirkulation wirken sich negativ auf die Daten- und Impulsweiterleitung und die Kraftübertragung aus, was dazu führen kann, dass Schmerzen an anderen Körperteilen entstehen. Vom Narbengewebe geht in der Regel eine Fernwirkung aus.

Die Kraftbeaufschlagung von Körpergewebe, insbesondere auch von Narbengewebe, kann manuell erfolgen, wobei ein aus Körpergewebe gebildeter Hautabschnitt beispielsweise zwischen Daumen und Zeigefinger eines Therapeuten mit Kraft beaufschlagt wird. Der Stand der Technik bietet bereits Lösungen an, wobei die manuelle und damit Schwankungen unterworfene Kraftbeaufschlagung durch mechanische Vorrichtungen ersetzt wird.

Aus der Druckschrift US 2007 009 3867 A1 ist bspw. ein Akkupressurclip mit zwei gegenüberliegenden Kontaktstellen bekannt, der bezüglich der zwischen Daumen und Zeigefinger befindlichen Daumenmuskulatur, speziell bezüglich Akkupressurpunkt L1-4 der Hand einer Person, angewendet wird.

Die Druckschrift US 5 666 964 zeigt eine Muskelbehandlungsvorrichtung mit Mess-, Krafteinstell- und Anzeigeeinrichtungen. Diese Vorrichtung ist im Sinne einer Messlehre mit Einstell- und Arretiermitteln und gegenüberliegenden Kontaktstellen ausgebildet. Die Anzeigeeinrichtung dient zur Anzeige der Messwerte des Dehnmessstreifens. Die Anwendung der bekannten Muskelbehandlungsvorrichtung richtet sich auf großformatige Muskeln des Wirbelsäulenbereiches. Trotzdem durch die Mess- und Anzeigemittel eine Kontrolle und damit eine Verbesserung der Behandlung im Hinblick auf die Berücksichtigung des Schmerzempfindens des Lebewesens möglich ist, besteht doch keine Möglichkeit der Datensammlung und Auswertung. Die bekannte Muskelbehandlungsvorrichtung erschöpft sich darin, gemäß dem persönlichen Schmerzempfinden des Lebewesens, die Klemmkraftbeaufschlagung zu unterbrechen. Die Anzeige der Klemmkraft gibt dem Therapeuten maximal Informationen darüber, wie stark die Beeinträchtigung des behandelten pathologischen Körpergewebes durch die Abweichung von Normalwerten eines gesunden Körpergewebes ist. Nachteilig ist außerdem, dass allein die subjektive Wahrnehmung des Lebewesens dazu führt, dass der Therapeut die Klemmkraft reduziert. Die subjektive Wahrnehmung kann von der Realität abweichen und es kann zu Beschädigungen des Körpergewebes kommen.

Weitere Gewebeeinwirkungsvorrichtungen entsprechend des Stands der Technik werden zum Beispiel in WO2012/106593A2, US2019/0117119A1 und US2011 /0082384 beschrieben.

Der Erfindung liegt die Aufgabe zugrunde, eine Vorrichtung zur Einwirkung auf das Körpergewebe anzugeben, die eine objektivierte Beurteilung des Körpergewebezustandes während der Kraftbeaufschlagung ermöglicht. Außerdem soll ein Verfahren zur Messung und Auswertung des Körpergewebezustandes unter Krafteinwirkung, insbesondere während des Einsatzes der hier in Rede stehenden Vorrichtung, angegeben werden.

Die voranstehende Aufgabe wird im Hinblick auf die Vorrichtung durch die Merkmale des Patentanspruches 1 gelöst. Danach ist eine Vorrichtung der in Rede stehenden Art derart ausgestaltet, dass eine zweite Messeinrichtung zur Erfassung des Abstandes zwischen den Kontakten vorgesehen ist, die mit der Anzeigeeinrichtung zusammenwirkt.

Die voranstehende Aufgabe wird im Hinblick auf das Verfahren durch die Merkmale des Patentanspruches 9 gelöst. Danach ist ein Verfahren der in Rede stehenden Art derart ausgestaltet, dass der Abstand zwischen den Kontakten gemessen wird.

Ausgehend von dem Stand der Technik gemäß US 5 666 964 ist im Hinblick auf die Vorrichtung und das Verfahren zunächst erfindungsgemäß erkannt worden, dass bisher nur die Klemmkraftwerte als Zahlenwerte auf der Anzeigeeinrichtung verfolgt wurden und in Kommunikation mit dem Patienten gemäß dessen subjektiven Schmerzempfinden die Klemmkraftbeaufschlagung gestoppt bzw. der Abstand der Klemmbacken vergrößert wurde.

Weiter ist erfindungsgemäß erkannt worden, dass das subjektive Schmerzempfinden eines Lebewesens oder dessen Rückäußerung zum Schmerzempfinden nicht immer den Grad der Einwirkung auf das Körpergewebe während einer Behandlung widerspiegeln muss. Insbesondere bei tierischen Lebewesen, kann per se keine verwertbare Rückkopplung zum Schmerzempfinden erfolgen.

Weiter ist erfindungsgemäß erkannt worden, dass unabhängig davon, ob es sich um gesundes oder verändertes Körpergewebe handelt, kaum gesicherte Erkenntnisse darüber existieren, welche Toleranz das Körpergewebe in den verschiedenen Körperregionen und den verschiedenen körperlichen Zuständen der Lebewesen im Hinblick auf eine Krafteinwirkung aufbringt.

Schließlich ist erfindungsgemäß erkannt worden, dass eine objektivierte Beurteilung des Körpergewebezustandes während der Kraftbeaufschlagung erreicht werden kann, wenn die Vorrichtung mit einer zweiten Messeinrichtung zur Erfassung des Abstandes zwischen den Kontakten / Klemmbacken ausgestattet ist, welche mit der Anzeigeeinrichtung zusammenwirkt und/oder wenn das Verfahren derart ausgestaltet ist, dass der Abstand zwischen den Kontakten gemessen wird.

Durch die zweite Messeinrichtung bzw. durch den zweiten Messvorgang wird mit dem Abstand der Kontakte eine konstante Größe zur Verfügung gestellt, die einen Vergleich aller über die Zeit folgenden Klemmkraftwerte mit der Anfangsklemmkraft ermöglicht. So kann die Zustandsänderung bzw. die Änderung der Gewebewiderstandskraft des eingeklemmten Körpergewebes nachvollzogen, ausgewertet und zum Vorteil des Lebewesens in eine Folgebehandlung einbezogen werden.

Für die erfindungsgemäße Vorrichtung und für das erfindungsgemäße Verfahren wird als wesentlich hervorgehoben, dass bisher weder gesundes noch krankhaftes Körpergewebe in seiner Reaktion auf Krafteinwirkungen intensiv untersucht wurde. Mit der erfindungsgemäßen Vorrichtung und dem erfindungsgemäßen Verfahren wird der Grundstein gelegt, Datenbanken anzulegen über Körpergewebsreaktionen bestimmter Körperregionen und bestimmter Zustände von Lebewesen. Gerade das Bindegewebe mit seinen Faszien und deren vielfältige Funktionen, wie bspw. die Kommunikation, die Reizweiterleitung zum Gehirn, das daraus die Lage des Körpers im Raum ableitet, oder die Kraftübertragung und -speicherung, die Dehnung von Muskeln oder das Halten von Muskelspannungen, ist noch weitgehend unerforscht. Durch die netzwerkartige Verbindung der Faszien des Bindegewebes untereinander wird eine objektivierte Messung und Auswertung des Körpergewebezustandes unter Krafteinwirkung wichtig im Hinblick auf die Erkennung von Ursachen für Schmerzen, die mehr oder weniger weit entfernt von dem behandelten Hautbereich auftreten können. Weiterführend können aus den Daten und Erkenntnissen Algorithmen erarbeitet und EDV-Programme geschrieben werden, die die Anwendung der Vorrichtung und des Mess- und Auswerteverfahrens verbessern und effizient gestalten.

Die Einstelleinrichtung zur Einstellung der Klemmkraft könnte vorzugsweise die zweite Messeinrichtung zur Messung des Abstandes zwischen den Kontakten umfassen. Zunächst könnten beide Kontakte zusammengefahren werden bis sie sich gerade berühren und so zunächst eine Nullmessung vorgenommen werden. Anschließend könnte der Hautabschnitt zwischen den wieder auseinander gefahrenen Kontakten positioniert werden. Nach einer besonders bevorzugten Ausführungsform könnte die bewegliche Klemmbacke auf einem Schenkel der feststehenden Klemmbacke geführt sein und zunächst im Sinne eines Schnellverschlusses in Richtung feststehende Klemmbacke verschoben und nach der Grobjustierung in einer Zahnleiste am Schenkel der feststehenden Klemmbacke über eine Rastnase einer Rasteinrichtung verrastet werden. Wenn dies abgeschlossen ist, kann eine Feinjustierung der ebenfalls in Bezug auf die Rasteinrichtung mit der Rastnase beweglichen Klemmbacke erfolgen, wobei über eine Einstellschraube der Abstand zur feststehenden Klemmbacke verringert wird und es zur Verklemmung des Hautabschnitts kommt. Wann eine Arretierung der Klemmeinrichtung stattfindet, bei welchem Abstand und bei welcher Anfangsklemmkraft könnte sich am sicheren Sitz des Hautabschnitts zwischen den Kontakten, an dem Empfinden des Lebewesens, an Werten für gesundes Körpergewebe und auch an den Erfahrungswerten des Therapeuten bzw. des Bedienpersonals der Vorrichtung ausrichten. Der Therapeut könnte bei der Bedienung der Einstellschraube den Gewebewiderstand der eingeklemmten Hautfalte erfassen und merken, wenn eine weitere Abstandsverringerung zwischen den Kontakten nicht mehr möglich ist. Hierbei könnte es dazu kommen, dass die bei der Feststellung des Abstandes herrschende Anfangsklemmkraft gleichzeitig den Maximalwert der Klemmkraft darstellt.

Der im Rahmen eines Zeitintervalls festgestellte Abstand der Kontakte und die Anfangsklemmkraft stehen in Korrelation, werden gemessen und für die Darstellung kommender Änderungen der Klemmkraft im Verhältnis zur Anfangsklemmkraft auf der Anzeigeeinrichtung benötigt. Der Hautabschnitt ist Bestandteil lebender Materie und innerhalb des mit Klemmkraft beaufschlagten Körpergewebes baut sich eine Gewebewiderstandskraft auf. Die Gewebewiderstandskraft wirkt der Klemmkraft entgegen - bspw. bei verklebten Faszien - und es kann zur betragsmäßigen Kräfteaddition und zum Anstieg der Klemmkraft kommen. Die Gewebewiderstandskraft könnte der Klemmkraft auch nachgeben - bspw. bei der Lösung von Verklebungen der Faszien - und es kann zur betragsmäßigen Kräftesubtraktion und zur Abnahme der Klemmkraft kommen. Das Nachlassen der Klemmkraft infolge nachlassender Gewebewiderstandskraft ist das gewünschte Ergebnis der ggf. wiederholten Anwendung der erfindungsgemäßen Vorrichtung und deren wiederholten Neueinstellung des Abstandes innerhalb eines Zeitintervalls. Die Klemmkrafteinstellung könnte manuell vorgenommen werden. Alternativ könnte auch ein Stellmotor angesteuert werden. Die Veränderung der Klemmkraft in Korrelation zur Gewebewiderstandskraft könnte auf der Anzeigeeinrichtung angezeigt werden. Besonders bevorzugt wird die Klemmkraft in Abhängigkeit von der Zeit bei einem vorgegebenen Abstand der Kontakte dargestellt. Der Zeitfaktor spielt eine große Rolle im Hinblick auf die Feststellung der Klemmkraftänderung in Form eines Anstieges oder einer Abnahme, einer Amplitude oder eines konstanten Verlaufes. Dabei spielen sowohl das Zeitintervall des Einstellens, also der Abstandsverringerung zwischen den Kontakten, als auch das Zeitintervall des Messens bei konstant eingestelltem Abstand eine Rolle. Bei beiden Zeitintervallen kann die Änderung der Klemmkraft in Korrelation zur Gewebewiderstandskraft auf der Anzeigeeinrichtung dargestellt und mitverfolgt werden.

Die Klemmeinrichtung der erfindungsgemäßen Vorrichtung könnte eine bewegliche und eine feststehende Klemmbacke aufweisen, deren Enden jeweils einen Kontakt ausbilden. Der feststehenden Klemmbacke der Klemmeinrichtung könnte die erste Messeinrichtung zur Erfassung der Klemmkraft zugeordnet sein. Die erste Messeinrichtung könnte einen druckempfindlichen Wägesensor oder einen Dehnmessstreifen umfassen, der die Klemmkraft erfasst. Die Klemmkraft könnte während der Klemmkraftbeaufschlagung des Hautabschnittes wegen dessen Gewebewiderstandskraft einer Änderung unterworfen sein, die den Zustand des verklemmten Körpergewebes widerspiegelt. Der beweglichen Klemmbacke der Klemmeinrichtung könnte die zweite Messeinrichtung zur Erfassung des Abstandes der Kontakte zugeordnet sein. Unter konstruktivem Aspekt könnten die erste und die zweite Messeinrichtung innerhalb einer Aussparung oder Aufnahme der jeweiligen Klemmbacke angeordnet sein. Alternativ könnten die erste und die zweite Messeinrichtung auch dem Kontakt zugeordnet sein. Bezüglich der zweiten, abstandsbezogenen Messeinrichtung besteht auch die Möglichkeit, diese der Einstelleinrichtung an der beweglichen Klemmbacke zuzuordnen.

Für den Anwender der erfindungsgemäßen Vorrichtung könnte ein Satz unterschiedlicher Klemmbacken mit den Kontakten für Hautabschnitte unterschiedlicher Abmessungen vorgehalten werden, die auswechselbar an der Klemmeinrichtung angeordnet werden können. Die Kontakte könnten mit Kontaktauflagen versehen sein, die bestimmte Formgebungen oder Oberflächen aufweisen, die besonders intensiv mit der Haut zusammenwirken. Zudem könnten die Kontakte und die Kontaktauflagen mehrteilig oder einteilig von unterschiedlicher Größe vorgehalten werden, so dass unterschiedliche Finger, mehrere Finger, einzelne Finger oder Daumen, ggf. auch Ellbogen oder weitere, auch von Therapeutenkörperteilen abweichende Formen simuliert werden können. Weiterführend könnten die Kontakte so beschaffen sein, dass sich die daran festzulegenden Kontaktauflagen in verschiedenen Richtungen positionieren lassen. Bspw. könnte der Kontakt eine kombinierte Schwenk-Arretier-Einrichtung aufweisen, wobei der Schwenkzapfen in eine Vertiefung an der Rückseite der Kontaktauflage unter Ausbildung einer lösbaren Verbindung eingreift. Gemäß einem besonders bevorzugten Ausführungsbeispiel könnte die erfindungsgemäße Vorrichtung im Sinne eines Bausatzes mit verschiedenen Bauteilen für verschiedene Anwendungen inklusive der für das Datenverarbeitungsgerät erforderlichen Software in einem Behälter zusammengefasst sein.

Nach einem bevorzugten Ausführungsbeispiel der erfindungsgemäßen Vorrichtung könnte eine Steuerung vorgesehen sein, die mit den Messeinrichtungen und der Anzeigeeinrichtung zusammenwirkt. Dabei könnte der Datentransfer elektrisch und / oder über Sender-Empfänger-Einheiten berührungslos stattfinden.

Die Steuerung könnte eine Datenverarbeitungsanlage in Form eines Laptops, Notebooks, Computers, Tablets, Mobiltelefons, Smartphones zur Verfügung stehen. Zusätzlich könnte eine Bedien- und Ableseeinheit vorgesehen sein, die einerseits mit der Klemmeinrichtung, andererseits mit der Datenverarbeitungsanlage gekoppelt sein könnte. Die Bedien- und Ableseeinheit könnte eine Aufbereitung der bei ihr ankommenden Messwerte für die Steuerung leisten. Außerdem könnte sie handlich gestaltet werden und einfach in das Gesichtsfeld eines Patienten verbracht werden, damit er die Anzeigeeinrichtung im Blick hat und die Klemmkraftänderung verfolgen kann. Wird nur mit einer Datenverarbeitungsanlage gearbeitet, so könnte diese in unmittelbarer Nähe der Klemmeinrichtung angeordnet sein, um einem menschlichen Patienten den Blick auf die Anzeigeeinrichtung zu ermöglichen.

Die Steuerung könnte Eingabemittel, Auswahlmittel und Speichermittel umfassen, um bspw. lebewesenbezogene Daten einzugeben und zu speichern, um bspw. eine Auswahl hinsichtlich der Art des Hautbereiches oder hinsichtlich des Behandlungsprofils für Fuß, Knie, Hüfte, Hand, Ellbogen, Schulter, Bandscheiben, Brust oder andere Körperteile zu treffen oder auch um bspw. eine Identifikation des Therapeuten, Datum und Uhrzeit einzugeben und zu speichern. Ein weiteres Kriterium, das eine wesentliche Rolle spielt, ist die Körpersymmetrie. Die Steuerung könnte ein Programm umfassen, das es ermöglicht, einen Vergleich von Messwerten vorzunehmen, die an zwei symmetrischen Körperbereichen ermittelt werden. Auf diese Weise kann es zu einer verbesserten, intensiven Analyse des Körpergewebezustandes kommen und schließlich zu einer Angleichung der Gewebewiderstandswerte im Zuge der Anwendung der Vorrichtung.

Vorzugsweise könnte die Steuerung der erfindungsgemäßen Vorrichtung verschiedene Daten einzeln oder in Kombination erfassen und verwerten sowie speichern und anderen Anwendungen und vergleichenden Auswertungen zugänglich machen. Bei der Anwendung der Vorrichtung auf pathologisches Körpergewebe oder Narbengewebe könnten insbesondere folgende Daten von Interesse sein:
- der fest eingestellte und von der zweiten Messeinrichtung erfasste Abstand der beiden Kontakte der Klemmeinrichtung und der Wert der bei diesem Abstand gemessenen Anfangsklemmkraft,
- die Zeitspanne, die benötigt wird, um Änderungen der Gewebewiderstandskraft des Körpergewebes herbeizuführen, insbesondere um das Körpergewebe zu lösen,
- die von der ersten Messeinrichtung erfassten Klemmkraftwerte in Korrelation zur sich ändernden Gewebewiderstandskraft des Körpergewebes,
- den Maximalwert der Klemmkraft in Korrelation zum Maximalwert der Gewebewiderstandskraft.

Die Zeitspanne könnte bezüglich des Anfangs durch den Zeitpunkt der Festlegung des Abstandes und Feststellung der Klemmbacken / Kontakte am Hautabschnitt und der dabei gemessenen Klemmkraft definiert sein. Bezüglich des Endes der Zeitspanne kommt ein Klemmkraftwert in Betracht, bei dem klar ist, dass die Klemmwirkung der Klemmbacken durch die abnehmende Gewebewiderstandskraft so nachgelassen hat, dass keine Klemmung mehr stattfindet. Dann könnte entweder ein neuer Vorgang mit verringertem Abstand der Kontakte eingeleitet oder die Anwendung beendet werden. Bei Nichtnachlassen der Klemmwirkung wird der Vorgang wegen Therapieresistenz abgebrochen.

Weitere Daten, die im Hinblick auf die Entwicklung eines automatisierten Therapieauswahlverfahrens, relevant sind, könnten betreffen:
- Daten zur Messstelle, wie Körperteil, Anordnung und Abmessung des Hautabschnittes,
- spezifische Daten zum Lebewesen, wie bspw. Schmerzempfindlichkeit, Alter, Geschlecht und Zustandsdaten des Lebewesens betreffend Herzfrequenz, Blutdruck, Körpergröße, Körpergewicht, Fettanteil.

Eine vorteilhafte Ausgestaltung der erfindungsgemäßen Vorrichtung könnte zur verbesserten Datenerfassung zur Beschaffenheit der Messstelle eine Beleuchtungseinrichtung umfassen. Die Beleuchtungseinrichtung könnte als LED vorliegen und den Hautabschnitt ausleuchten.

Des Weiteren könnten die Werte einer auf den Hautabschnitt aufgebrachten Zugkraft in die Steuerung eingegeben werden. Die Steuerung könnte auch Informationen über verfügbare und geeignete Kontaktaufsätze zur Variation der Kontaktfläche je nach Anforderung für den zu behandelnden Hautbereich umfassen. In die Steuereinrichtung könnten auch die Werte der Klemmkraftänderungsprofile in Korrelation zur Gewebewiderstandskraftänderung von Hautabschnitten an symmetrischen Körperteilen eingegeben werden und dort ausgewertet werden.

Die hier angegebenen Beispiele für die Daten sind nicht abschließend. Insbesondere die Einbeziehung symmetrischer Körperteile zu Zwecken der Vergleichsmessung und weiteren Analyse bis hin zur Angleichung der Gewebewiderstandszustände bei symmetrischen Körperteilen eröffnet weitere Anwendungsmöglichkeiten der erfindungsgemäßen Vorrichtung. Weiterführend könnten auch benachbarte Organe untersucht, datentechnisch erfasst und verglichen sowie ausgewertet werden.

Gemäß einer besonders bevorzugten Ausgestaltung der erfindungsgemäßen Vorrichtung könnte diese eine Sicherheitstoppeinrichtung umfassen, die durch die Steuerung und / oder durch das menschliche Lebewesen auslösbar ist, wenn ein bestimmter Abstand der Kontakte der Klemmeinrichtung unterschritten bzw. ein bestimmter Klemmkraftbetrag überschritten wird und / oder wenn ggf. ein bestimmter Zugkraftbetrag überschritten wird. Grade bei tierischen Lebewesen, die aktiv keinen "Notknopf" auslösen können, ist die Anzeigeeinrichtung mit den Informationen zur Klemmkraft in Korrelation zur Gewebewiderstandskraft außerordentlich hilfreich, um unnötige Schmerzen zu vermeiden.

Damit der Therapeut oder der Patient bzw. das Lebewesen direkt optisch oder akustisch, ggf. auch durch Vibration alarmiert wird, wenn eine Zustandsänderung des Körpergewebes und dessen Gewebewiderstandskraft unter Krafteinwirkung stattfindet, könnte die erste Messeinrichtung zur Erfassung der Klemmkraft eine Signaleinrichtung umfassen oder mit einer bspw. in einer Datenverarbeitungsanlage oder Bedieneinheit enthaltenen Signaleinrichtung zusammenwirken. Die Signaleinrichtung könnte verschiedene Zustände signalisieren, insbesondere:
a) die Verminderung der Klemmkraft in Korrelation zur Gewebewiderstandskraft nach Erreichen eines Maximalwertes und
b) das Gleichbleiben der Klemmkraft in Korrelation zur Gewebewiderstandskraft insbesondere nach Erreichen eines Maximalwertes.

Im Fall a) führt dies dazu, dass der Abstand zwischen den Kontakten der Klemmeinrichtung verringert und damit der Wert der aufgebrachten Klemmkraft am Anfang einer Zeitspanne erhöht wird. Im Fall b) führt dies zum Stoppen der Krafteinwirkung auf das Körpergewebe. Der konstante Wert kann sich auf dem Niveau des Maximalwertes einstellen, ggf. aber auch auf einem niedrigeren Niveau.

Die Signaleinrichtung könnte mit der Anzeigeeinrichtung oder Lautsprechern eines Computers, der die Steuerung umfasst oder auch mit einer separaten, am Körper des Lebewesens und/oder Therapeuten angeordneten Vibrationsalarmeinrichtung gekoppelt sein.

Eine weitere vorteilhafte Ausgestaltung der erfindungsgemäßen Vorrichtung sieht vor, die Klemmeinrichtung einer Halteeinrichtung, insbesondere in Form eines Stativs, zuzuordnen. Die Halteeinrichtung erleichtert oder ersetzt das Halten der Klemmeinrichtung durch den Therapeuten komplett. Auf diese Weise wird der Verfälschung von Messergebnissen entgegengewirkt, es wird Kraft eingespart und der Therapeut könnte sich bereits - im Falle des Vorhandenseins einer zuvor beschriebenen Signaleinrichtung - auch einer anderen Tätigkeit zuwenden. Damit die Klemmeinrichtung bequem an der zutreffenden Körperregion angesetzt werden kann, könnten die Halteeinrichtung und / oder die Klemmeinrichtung vorzugsweise verschiedene Befestigungsmittel umfassen, die die Anordnung der Klemmeinrichtung in verschiedenen Positionen, wie frontal oder sagittal, in Bezug zum Lebewesen erlauben. Alternativ oder zusätzlich könnten die Kontaktauflagen drehbar an den Kontakten angeordnet sein und so die Positionierung zum Hautabschnitt oder zur Hautfalte optimieren.

Die erfindungsgemäße Vorrichtung könnte dahingehend vorteilhaft fortgebildet sein, dass sie eine Zugeinrichtung umfasst, mittels der eine Zugkraft auf die Klemmeinrichtung und den darin eingeklemmten Hautabschnitt ausübbar ist. Die Krafteinwirkung auf den Hautabschnitt mittels einer Zugkraft könnte im Hinblick auf die Narbengewebebehandlung besonders vorteilhaft sein. Das Narbengewebe erstreckt sich in der Regel multidirektional zu den Faszien und verursacht dadurch Spannungen. Durch eine von außen auf die Haut aufgebrachte Zugkraftbeaufschlagung kann eine Dehnung und venöse Entstauung des Fasziengewebes erzielt werden. Im Hinblick auf die Behandlung des Narbengewebes könnte auf die aus der Boeger^{®} Therapie bekannte manuelle Lösetechnik zurückgegriffen werden, wobei Narbengewebe mittels Daumen und einem Finger (Zeige- oder Mittelfinger) eine Zange ausgebildet und der Hautabschnitt in Form einer Hautfalte im Bereich der Narbe angehoben wird. Durch die erfindungsgemäße Vorrichtung wird der subjektive Faktor des Therapeuten verringert und die Spannung im Narbengewebe und im Fasziengewebe herabgesetzt.

Vorzugsweise könnte eine dritte Messeinrichtung zur Erfassung der aufgebrachten Zugkraft vorgesehen sein. Zur Realisierung der Zugkraftbeaufschlagung könnte die Halteeinrichtung ein Stativ mit einem Seilzug und einer Sperreinrichtung aufweisen. Die Klemmeinrichtung könnte am Ende des Seilzuges höhenverstellbar angeordnet sein. Damit der Hautbereich beim Aufbringen der Zugkraft zwischen den Kontakten der Klemmbacken der Klemmeinrichtung verbleibt, könnten besondere Kontaktauflagen mit besonders haftenden Oberflächen vorgesehen sein. Hier könnte mit Strukturen und Gummiprofilen gearbeitet werden. Besonders bevorzugt ist die Kombination dieser zugkraftbezogenen Fortbildung der erfindungsgemäßen Vorrichtung mit der Steuerung, so dass alle Krafteinwirkungen und Körperzustandsänderungen erfasst und ausgewertet werden können. Ausgehend von allen Daten könnten Löseprogramme bzw. Algorithmen mit Nachstellzyklen betreffend die auf den Hautabschnitt einwirkende Kraft - sowohl der Klemmkraft als auch der Zugkraft - für spezielle Körperregionen und Hautabschnitte sowie Patiententypen erstellt werden.

Eine weitere Fortbildung der erfindungsgemäßen Vorrichtung könnte eine Wärmebildkamera umfassen, um Veränderungen, wie bspw. Stauungen oder Homogenisierungen, Verklebungen im Körpergewebe, im interessierenden Hautabschnitt darzustellen. Weiterführend könnte die Vorrichtung einen Temperaturmesssensor aufweisen, der die gemessene Temperatur an die Steuerung bzw. die die Steuerung enthaltende Datenverarbeitungsanlage überträgt. Ein in der Steuerung bzw. Datenverarbeitungsanlage enthaltenes Programm könnte die Temperaturwerte und die Klemmkraftwerte entsprechend den Körpergewebezuständen verknüpfen und auch in Beziehung zu allen anderen Daten setzen. Es wurde festgestellt, dass pathologisches Körpergewebe eine gegenüber gesundem Gewebe veränderte Temperatur aufweist. Durch die Anwendung einer derart weitergebildeten erfindungsgemäßen Vorrichtung ergibt sich die Möglichkeit, die Temperaturänderung als Anzeichen des Erfolges der Behandlung auszuwerten. Auch hier könnte ein Kontrollsystem eingerichtet werden, das bei unerwarteter Temperaturänderung warnt.

Die Vorrichtung könnte des Weiteren eine Serotonintesteinrichtung umfassen, die das Blut oder den Urin, besonders einfach aber den Speichel des Lebewesens auf Serotonin vor und nach der Krafteinwirkung auf den Hautabschnitt misst. Hieraus lässt sich der Stressfaktor vor und nach der Behandlung und deren Erfolg ableiten.

Der Vorrichtung könnten noch diverse zusätzlichen Messeinrichtungen und Funktionseinrichtungen zugeordnet sein im Hinblick auf die Herzfrequenz, die Atemfrequenz, die Temperatur, eine Memory-Funktion für Anwendungen, das Datum, die Uhrzeit, die Gesamtdauer, mögliche Unterbrechungen, die Anzahl der Klemmkraftbeaufschlagungen und die Steigerungsrate in Prozent.

Die im Hinblick auf die Vorrichtung erläuterten Vorteile gelten grundsätzlich auch für das erfindungsgemäße Mess- und Auswerteverfahren.

Das erfindungsgemäße Verfahren könnte auf gesundes oder pathologisches Körpergewebe angewendet werden. Bei der Messung am gesunden Körpergewebe könnte eine Belastungstoleranz ermittelt werden, aus der Vorgaben über Kraft- und Zeitintervalle abgeleitet werden können, die dann im Hinblick auf pathologisches Körpergewebe eine Rolle spielen.

Wie bei der erfindungsgemäßen Vorrichtung ist es auch bei dem erfindungsgemäßen Verfahren für den Therapeuten und für den Patienten von allergrößtem Vorteil, dass die Änderung der Klemmkraft in Korrelation zur Gewebewiderstandskraft während der Klemmkraftbeaufschlagung über die Zeit gemessen und auf der Anzeigeeinrichtung dargestellt wird. Durch die Erfassung der Klemmkraft über die Zeit können Maxima oder Minima, Anstieg oder Abnahme oder Konstanten der Klemmkraftwerte in Korrelation zur Gewebewiderstandskraft des den Zustand ändernden Körpergewebes generiert und auf der Anzeigeeinrichtung dargestellt werden. Durch die Darstellung der Zustandsänderung in Form einer mathematischen Funktion, einer Kurve, eines Diagrammes in der Anzeigeeinrichtung, insbesondere während eines Zeitintervalls, wobei die Kontakte festgestellt sind und einen konstanten Abstand aufweisen, kann zunächst grundsätzlich erkannt werden, ob die Klemmkrafteinwirkung zu einem Therapieeffekt führt oder nicht. Je nachdem, ob ein Therapieeffekt erkannt wird oder nicht, kann weiterführend die Abstandseinstellung und Klemmkraftbeaufschlagung fortgesetzt oder gestoppt werden. Die subjektive Schmerzempfindung des Lebewesens wird bei der Entscheidung zum Stopp der Klemmkraftbeaufschlagung bzw. zum Auseinanderfahren der Kontakte berücksichtigt.

Bezüglich des erfindungsgemäßen Verfahrens wird als besonders vorteilhaft hervorgehoben, dass ein Maximalwert der Klemmkraft in Korrelation zur Gewebewiderstandskraft ermittelt werden könnte und dass der Maximalwert dahingehend ausgewertet werden könnte, ob er eine Amplitude oder einen konstanten Verlauf aufweist. Bei der Feststellung eines als Amplitude vorliegenden Maximalwertes der Klemmkraft in Korrelation zur Gewebewiderstandskraft könnte eine Neueinstellung des Abstandes der Klemmbacken erfolgen. Bei der Feststellung eines konstanten Maximalwertes der Klemmkraft in Korrelation zur Gewebewiderstandskraft könnte die Klemmkraftbeaufschlagung gestoppt werden.

Der Maximalwert der Klemmkraft in Form einer Amplitude und die auf das Maximum folgenden geringeren Klemmkraftwerte könnten Informationen zum Fortschreiten der Lösung von Körpergewebe sowie zum Schmerzabbau während und nach der Lösung des Körpergewebes liefern.

Wenn eine Konstante der Klemmkraft in Korrelation zur Gewebewiderstandskraft festgestellt wird, könnten daraus Informationen zur Therapieresistenz gewonnen werden. Eine Erkenntnis könnte sein, dass es sich um gesundes Körpergewebe handelt. Eine andere Erkenntnis könnte sein, dass sich Spannungen im behandelten Hautabschnitt überlagern und so die Therapie verhindern. Über weitere erfahrungsgemäß mit dem behandelten Hautabschnitt in Verbindung stehende Narben enthaltende Hautabschnitte könnte die Spannungsüberlagerung aufgelöst werden und der ursprünglich behandelte Hautabschnitt könnte dann keine Therapieresistenz mehr zeigen.

Die aus der manuellen Körpergewebebehandlung bekannte Lehre, dass gelöstes Körpergewebe gelöst bleibt, könnte gemäß einer Weiterbildung des Verfahrens dadurch überprüft werden, dass im Fall eines erfolgreichen Schmerzabbau eine Kontrollmessung der Klemmkraft in Korrelation zur Gewebewiderstandskraft durchgeführt wird. Die "Lösung" von Körpergewebe bedeutet, dass dortige Verklebungen oder Stauungen infolge Krafteinwirkung auf das Körpergewebe beseitigt sind. Im besonderen Fall des Narbengewebes kann "Lösen" auch bedeuten, dass Narbengewebe auseinandergezogen oder auseinandergerissen wird und auf diese Weise Spannungen beseitigt werden, so dass die Flüssigkeitszirkulation insgesamt verbessert wird.

Gemäß einer besonders bevorzugten Ausgestaltung des erfindungsgemäßen Verfahrens könnten mindestens zwei aus Körpergewebe symmetrischer Körperteile gebildete Hautabschnitte mit Klemmkraft beaufschlagt werden und die gemessenen Klemmkraftwerte in Korrelation zur Gewebewiderstandskraft miteinander verglichen werden. Die verschiedenen Hautabschnitte könnten nacheinander oder zeitgleich mit zwei verschiedenen Vorrichtungen mit Klemmkraft beaufschlagt werden. Dabei kann über Beweglichkeitstests die Reihenfolge oder Zeitgleichheit der Behandlung festgelegt werden. Jedenfalls ist bei symmetrischen Körperteilen und dortigen Hautabschnitten zumeist eines in besserem Zustand als das andere. Hieraus können Schlussfolgerungen für die Belastungstoleranz bzw. Schmerzempfindlichkeit gezogen werden. Bei einem Vergleich der beiden gemessenen Klemmkraftwerte in Korrelation zu den Gewebewiderstandswerten mit denen gemäß einer Datei, die auf der Grundlage von gesundem Körpergewebe angelegt wurde, könnte bspw. festgestellt werden, dass keiner der beiden Hautabschnitte der beiden symmetrischen Körperteile eine gesunde Belastungstoleranz hat, sondern dass sie nur unterschiedlich stark schmerzempfindlich sind. Die fortwährende Messung der Klemmkraft in Korrelation zur Gewebewiderstandskraft und die Nachstellung des Abstandes und die damit verbundene Änderung der Anfangsklemmkraft eines nächsten Zeitintervalls könnte dazu führen, dass der vormals schmerzempfindlichere Hautabschnitt eine größere Belastungstoleranz erreicht als der vormals weniger schmerzempfindliche Hautabschnitt. Dann könnten beide Vergleichshautabschnitte auf ein gemeinsames Lösungs-Niveau gebracht werden und so eine Balance zwischen den Körperhälften hergestellt werden. Die auf Hautabschnitte an symmetrischen Körperteilen bezogenen Messungen, Vergleiche und Auswertungen könnten auch im Hinblick auf benachbarte Organe ohne Symmetrieentsprechung angewendet werden. Bspw. könnte die Klemmkraft in Korrelation zur Gewebewiderstandskraft von Hautabschnitten in Bereich der Leber einerseits und im Bereich des Darms andererseits miteinander in Beziehung gesetzt werden.

Bei menschlichen Lebewesen könnte parallel zur Messung und Anzeige der Klemmkraftwerte während der Klemmkrafteinwirkung eine Schmerzabfrage durchgeführt werden. Die gilt auch bei der Zugkraftbeaufschlagung. Die Schmerzabfrage könnte vom Therapeuten oder durch ein Computerprogramm bzw. eine Abfragesoftware vorgenommen werden. Die Schmerzabfrage geht einher mit der Kraftbeaufschlagung und der Ausbildung des Maximalwertes in Form einer Amplitude oder eines konstanten Wertes.

In bestimmten Fällen der unzutreffenden Schmerzwahrnehmung, dahingehend, dass der Patient noch keinen Schmerz empfindet, obwohl die Klemmkraft in Korrelation zur Gewebewiderstandskraft ansteigt und der Schmerz erfahrungsgemäß zunehmen müsste, könnte das erfindungsgemäße Verfahren dahingehend weitergebildet sein, dass die Schmerzangaben des menschlichen Patienten zu dessen Schutz vor unzutreffender Wahrnehmung kontrolliert werden, indem vor und während der Krafteinwirkung ermittelte Werte betreffend den Blutdruck und / oder die Herzfrequenz miteinander verglichen und erforderlichenfalls ein Abschalten der Vorrichtung herbeigeführt wird. Zu den unzutreffenden Schmerzwahrnehmungen und damit zur fehlerhaften Information während der Schmerzabfrage kann es aufgrund neurologischer Störungen kommen oder dadurch, dass der Patient den Schmerz aktiv unterdrückt. Über den Blutdruckvergleich und / oder den Herzfrequenzvergleich wird die subjektive Aussage des Patienten kontrolliert und es kommt zu einer weiteren Objektivierung der Beurteilung des Körpergewebes. Alternativ zu einem Vergleich patientenspezifischer Daten könnte auch auf einen Vergleichswert betreffend den Blutdruck und die Herzfrequenz vor Krafteinwirkung zurückgegriffen werden, der aus einer Datenbank stammt, in der der Patiententypus mit all seinen physiologischen Eigenschaften gespeichert ist, dem der Patient entspricht.

Ausgehend von der Erkenntnis, dass pathologisches Körpergewebe eine gegenüber gesundem Gewebe veränderte Temperatur aufweist, könnte das erfindungsgemäße Verfahren dahingehend weitergebildet sein, dass vor, ggf. während und nach der Krafteinwirkung ermittelte Temperaturwerte im Bereich des Hautabschnitts miteinander verglichen werden. Hieraus könnte - im Falle der Temperaturänderung nach der Behandlung - abgeleitet werden, dass sich der Körpergewebezustand verbessert, gelöst hat und sich die Belastungstoleranz wieder an die von gesundem Körpergewebe annähert. In diesem Zusammenhang wird die Wichtigkeit der Anwendung des Verfahrens auch auf gesundes Körpergewebe hervorgehoben, wodurch eine Vergleichsdatenbank überhaupt erst möglich wird. Der Verfahrensschritt des Temperaturvergleichs könnte dahingehend weitergebildet werden, dass die Temperaturwerte nicht nur miteinander verglichen werden, sondern durch eine entsprechend gestaltete Steuerung bzw. Software in Beziehung zu weiteren Daten gesetzt werden. Solche Daten könnten die gemessenen Klemm- oder Zugkräfte in Korrelation zu den Gewebewiderstandskräften des Körpergewebes und die physiologischen spezifischen Eigenschaften des Patienten betreffen. Die Daten eines Patienten und deren Verknüpfungen können in Datenbanken eingepflegt werden und es kann zur Feststellung von Patiententypen kommen. Aus ähnlichen Daten und ähnlichen Verknüpfungen lassen sich Schlussfolgerungen ableiten, spezielle Patiententypen aufzufinden und schließlich das erfindungsgemäße Verfahren zu automatisieren und den Anteil des Therapeuten an der Behandlung zu minimieren.

Zusätzlich oder alternativ zur Temperaturmessung könnten auch die Serotoninwerte des Lebewesens vor und nach der Krafteinwirkung ermittelte und miteinander verglichen werden. Hieraus lässt sich der Stressfaktor vor und nach der Behandlung und deren Erfolg ableiten. Vorzugsweise enthält die Vorrichtung auch eine Serotonintesteinrichtung, deren Probematerial vorzugsweise der Speichel des Lebewesens ist.

Das erfindungsgemäße Mess- und Auswerteverfahren birgt ein hohes Potential im Hinblick auf eine Erhöhung der Effizienz bei der objektivierten Feststellung des Körpergewebezustandes. Insbesondere könnten in vorteilhafter Weise zum Zweck einer Verallgemeinerungsauswertung Datensammlungen zu Zykluszeiten der Krafteinwirkung beim Einstellen oder bei konstantem Kontakabstand, Wiederholungen der Krafteinwirkungen an ähnlichen Hautabschnitte von Lebewesen mit ähnlichen Eigenschaften und ähnlichem pathologischen Körpergewebe angelegt und zur Erstellung von Algorithmen verwendet werden, die das Mess- und Auswerteverfahren weiter verselbstständigen. Die Verwendung künstlicher Intelligenz ist gerade im Hinblick auf die Unterbrechung der Kraftbeaufschlagung entweder zur Erreichung eines Stopps, oder zur Neueinstellung des Abstandes der Klemmbacken der Vorrichtung, ggf. die Höhenveränderung des Seilzuges der Vorrichtung, eine weitere Option.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung von Ausführungsbeispielen der Erfindung anhand der Zeichnung zu verweisen. In Verbindung mit der Erläuterung der Ausführungsbeispiele der Erfindung werden auch im Allgemeinen bevorzugte Ausgestaltungen und Weiterbildungen der Lehre erläutert. In der Zeichnung zeigen
- Fig. 1: in schematischer skizzenhafter Darstellung, ein erstes Ausführungsbeispiel der erfindungsgemäßen Vorrichtung,
- Fig. 2: in schematischer skizzenhafter Darstellung ein vergrößertes Detail aus Fig. 1,
- Fig. 3: in schematischer skizzenhafter Darstellung, ein zweites Ausführungsbeispiel der erfindungsgemäßen Vorrichtung,
- Fig. 4: in schematischer skizzenhafter Darstellung ein vergrößertes Detail aus Fig. 3,
- Fig. 5: in schematischer skizzenhafter Darstellung einen Hautabschnitt, der an Ober- und Unterseite mit Kraft beaufschlagt wird,
- Fig. 6: in schematischer skizzenhafter Darstellung einen Hautabschnitt, in Form einer Hautfalte,
- Fig. 7: in schematischer Darstellung eine Steuereinrichtung der erfindungsgemäßen Vorrichtung gemäß einem ersten Ausführungsbeispiel,
- Fig. 8: in schematischer Darstellung eine Steuereinrichtung der erfindungsgemäßen Vorrichtung gemäß einem zweiten Ausführungsbeispiel,
- Fig. 9: ein Fließschema für ein Ausführungsbeispiel des Verfahrens und
- Fig. 10: eine vereinfachte Darstellung eines Diagramms infolge Messung und Auswertung einer Behandlung an einem im Bereich einer Impfnarbe des rechten Oberarmes ausgebildeten Hautabschnittes eines menschlichen Patienten im Rahmen einer Anwendung der erfindungsgemäßen Vorrichtung und des Verfahrens.

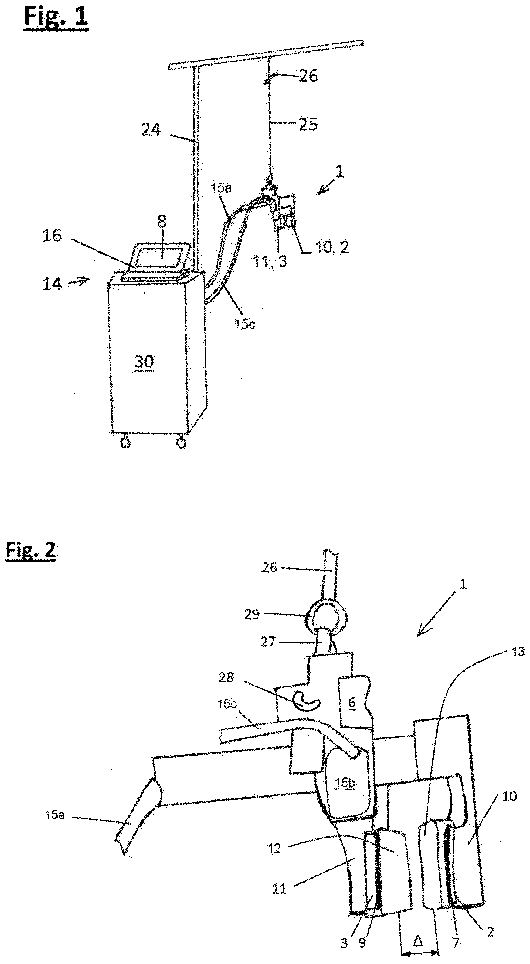

Die Figuren 1 und 2 zeigen eine Vorrichtung zur Einwirkung auf das Körpergewebe, eines Lebewesens mit einer Klemmeinrichtung 1 mit voneinander beabstandeten Kontakten 2, 3 zum Einklemmen eines aus Körpergewebe gebildeten Hautabschnitts 4, 5.

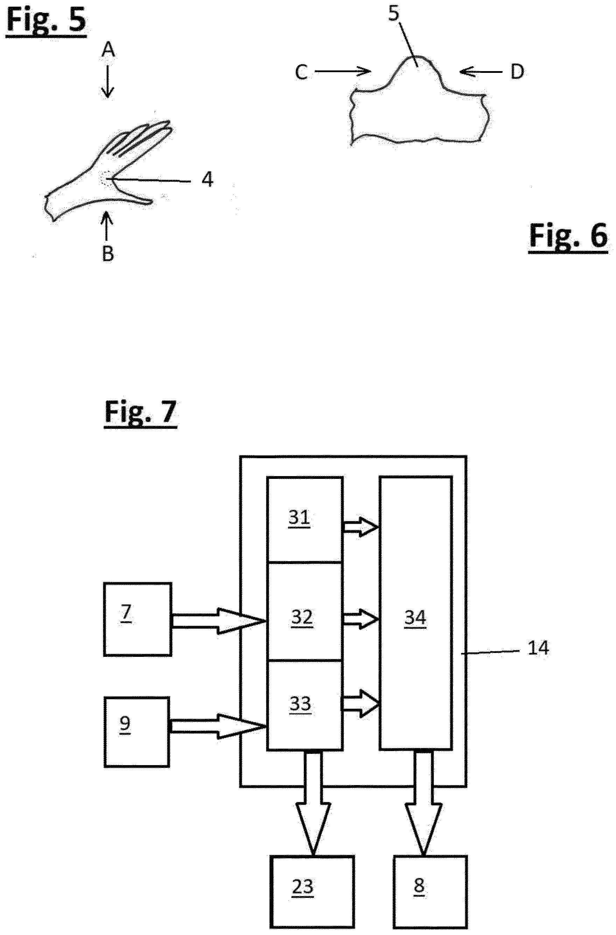

Die hier relevanten zwei Arten von Hautabschnitten 4, 5 sind in den Fig. 5 und 6 gezeigt. Der Hautabschnitt 4 in Fig. 5 betrifft einen mit gestrichelter Linie markierten Bereich zwischen Daumen und Zeigefinger. Bei diesem Hautabschnitt wird die Klemmeinrichtung 1 an Ober- und Unterseite der Hand im betreffenden Bereich des Hautabschnittes 4 entsprechend den Pfeilen A und B angesetzt, mit dem Ziel, auf den Daumenmuskel einzuwirken. Der Hautabschnitt 5 liegt in Form einer Hautfalte vor, welche Narbengewebe enthält. Die Klemmeinrichtung 1 setzt bei dem Hautabschnitt 5 etwa in der Mitte der Hautfalte an. Die Krafteinleitung erfolgt bei Hautabschnitt 5 gemäß den Pfeilen C, D.

Die Klemmeinrichtung 1 umfasst gemäß Fig. 2 eine schematisch gezeigte Einstelleinrichtung 6 mit einer Einstellschraube zur Einstellung der auf das Körpergewebe aufgebrachten Klemmkraft FK, mit einer ersten Messeinrichtung 7 zur Ermittlung der auf den Hautabschnitt 4, 5 aufgebrachten Klemmkraft FK.

Des Weiteren ist eine in den Fig. 1, 3 und 4 gezeigte Anzeigeeinrichtung 8 zur Darstellung der Messwerte der Messeinrichtung 7 vorgesehen.

Erfindungsgemäß ist die Vorrichtung mit einer zweiten Messeinrichtung 9 zur Erfassung des Abstandes Δ zwischen den Kontakten 2, 3 ausgestattet, welche mit der Anzeigeeinrichtung 8 zusammenwirkt. Auf der Anzeigeeinrichtung 8 wird die Veränderung der Klemmkraft FK in Korrelation zur Gewebewiderstandskraft des Hautabschnitts 4, 5 in Abhängigkeit von der Zeit t während der Einstellung und bei einem vorgegebenen Abstand Δ dargestellt.

Die Klemmeinrichtung 1 weist eine feststehende Klemmbacke 10 und eine bewegliche Klemmbacke 11 auf, an denen sich die Kontakte 2, 3 befinden. Die Kontakte 2, 3 sind mit Kontaktauflagen 12, 13 versehen. Die erste Messeinrichtung 7 zur Erfassung der Klemmkraft FK ist der feststehenden Klemmbacke 10 zugeordnet und ist in Fig. 2 vereinfacht, schematisch durch eine schwarze schmale Fläche dargestellt. Die zweite Messeinrichtung 9 zur Erfassung des Abstandes Δ zwischen den Kontakten 2, 3 ist der beweglichen Klemmbacke 11 zugeordnet und ist ebenfalls vereinfacht, schematisch durch eine schwarze schmale Fläche dargestellt.

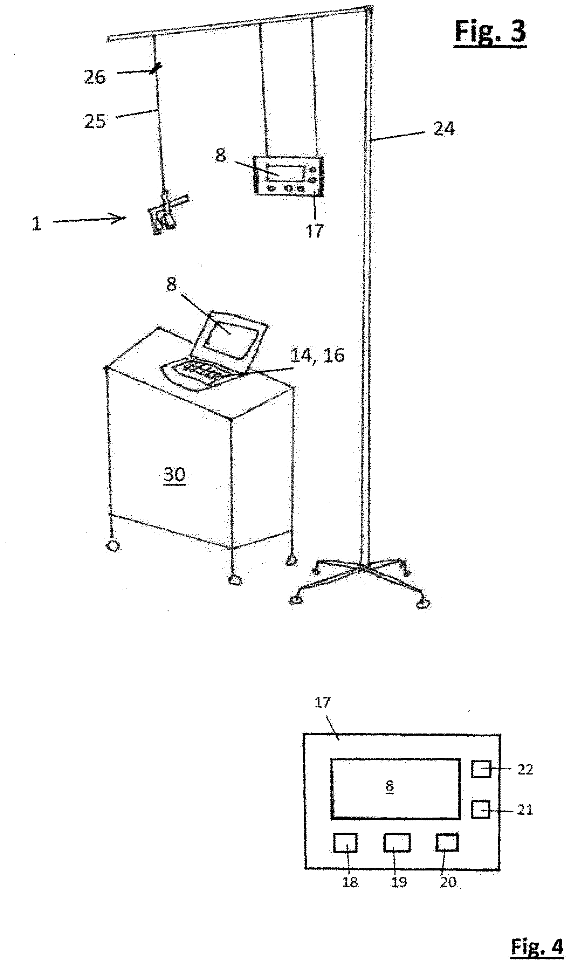

Die Figuren 1 und 3 zeigen zwei verschiedene Ausführungsbeispiele der Vorrichtung, die beide eine Steuerung 14 umfassen, die mit den Messeinrichtungen 7, 9 und der Anzeigeeinrichtung 8 zusammenwirkt. Bei dem ersten Ausführungsbeispiel gemäß Fig. 1 sind die Messeinrichtungen 7, 9 über elektrische Leitungen 15a und 15c mit der in einem Datenverarbeitungsgerät 16 in Form eines Laptops 16 enthaltenen Steuerung 14 verbunden, wodurch der Datentransfer gewährleistet ist. Dabei ist die erste Messeinrichtung 7 mit der Leitung 15a verbunden. An der beweglichen Klemmbacke 11 befindet sich ein Messwerteaufbereitung 15b, die die von der zweiten Messeinrichtung 9 kommenden Messwerte betreffend den Abstand Δ zwischen den Kontakten 2, 3 aufbereitet und dann über die elektrische Leitung 15c zur Steuerung 14 sendet.

Bei dem zweiten Ausführungsbeispiel gemäß Fig. 3 ist die Steuerung 14 ebenfalls im Datenverarbeitungsgerät 16 in Form eines Laptops 16 enthalten. Mit 17 ist eine Bedieneinheit bezeichnet, die zwar auch ein Datenverarbeitungsgerät ist, jedoch ohne Steuerung. Das Laptop 16 enthält Vergleichsdatenbanken und Software mit verschiedenen Auswertealgorithmen und hat Zugriff und Speichermöglichkeit für sämtliche Daten der Bedieneinheit 17, die eine nicht näher bezeichnete Messwerteaufbereitung umfasst und an das Laptop 16 weiterleitet.

Das als handliche Bedieneinheit 17 vorliegende Datenverarbeitungsgerät 17 gibt dem Patienten die Möglichkeit, während der Behandlung die Darstellung der Klemmkraft FK in Korrelation zur Gewebewiderstandskraft auf der dortigen Anzeigeeinrichtung 8 bequem mitzuverfolgen. Bei dem zweiten Ausführungsbeispiel sind die beiden Datenverarbeitungsgeräte 16, 17 und die Messeinrichtungen 7, 9 über hier nicht dargestellte Sender-Empfänger-Einheiten zur Realisierung des Datentransfers berührungslos verbunden. Zusätzlich zum Erhalt aufbereiteter Messwerte von der Bedieneinheit 17 hat der Laptop 16 redundant auch Zugriff zu den Rohmessdaten der beiden Messeinrichtungen 7, 9.

Die Steuerung 14 umfasst über die Tastatur des Laptops 16 Eingabemittel, Auswahlmittel und Speichermittel. Beim zweiten Ausführungsbeispiel kommen die über Sender-Empfänger-Einheiten mit dem Laptop 16 berührungslos verbundenen Bedienelemente 18, 19, 20, 21, 22 der Bedieneinheit 17 als Eingabemittel, Auswahlmittel und temporäre Speichermittel hinzu.

Das Bedienelement 18 der Bedieneinheit 17 hat eine Snapshotfunktion / Momentaufnahmefunktion und ist ein Speichermittel, um die aktuelle Darstellung der Klemmkraft FK in Korrelation zur Gewebewiderstandskraft des behandelten Hautabschnittes 4, 5 auf der Anzeigeeinrichtung 8 der Bedieneinheit 17 und des Laptops 16 festzuhalten. Die tatsächliche Speicherung der Snapshot-Darstellung findet im Speicher des Laptops 16 statt. Da die Bedieneinheit 17 gemäß dem zweiten Ausführungsbeispiel eine geringe Speicherkapazität hat, ist das Bedienelement 19 ein Eingabemittel mit einer Löschfunktion für die Bedieneinheit 17 versehen. Das Bedienelement 20 ist ebenfalls ein Eingabemittel mit einer Resetfunktion zum Zurücksetzen auf Start, die dann benutzt wird, wenn nach dem ersten Messvorgang der Abstand Δ der Klemmbacken 10, 11 bzw. der Kontakte 2, 3 verringert wird. Die Bedienelemente 21 und 22 betreffen Auswahlmittel zur Anzeige des Abstandes Δ zwischen den Kontakten 2, 3 und zur Anzeige der Klemmkraft FK, die beim Klemmen des Hautabschnittes 4, 5 in Korrelation zur Gewebewiderstandskraft des Körpergewebes gemessen wird. Zusätzlich wird bei Bedienelement 22 eine Eingabemöglichkeit für den subjektiv empfundenen Schmerz S bei menschlichen Patienten vorgesehen.

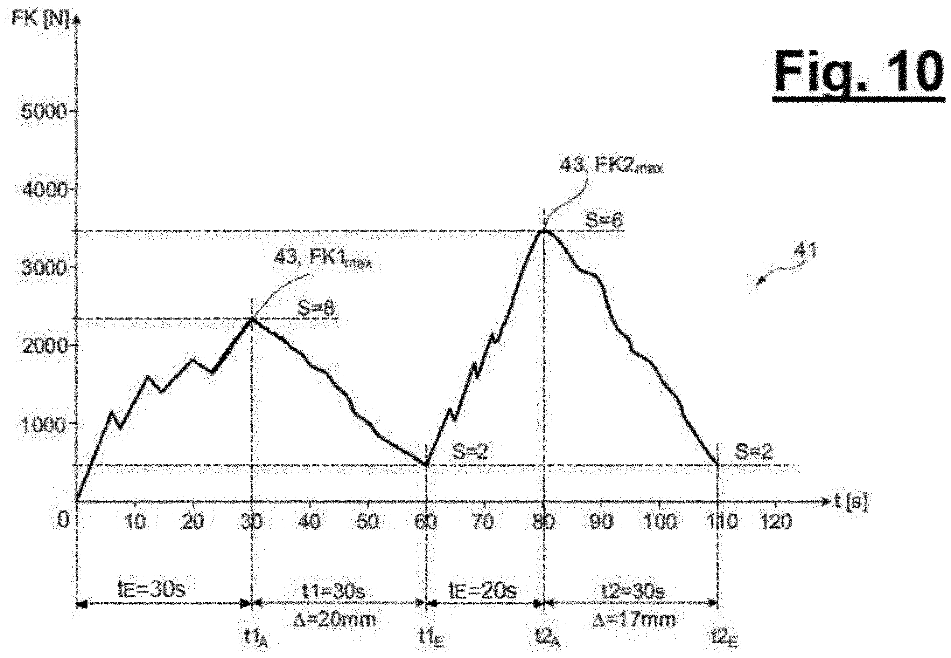

Der Schmerz S wird auf einer numerischen Schmerzbewertungsskala mit maximal zehn Schmerzeinheiten eingeordnet und wird gemäß dem in Fig. 10 dargestellten Ausführungsbeispiel in einem Diagramm 41 in Bezug zur Klemmkraft FK in der Anzeigeeinrichtung 8 dargestellt. Der Wert 10 der numerischen Schmerzbewertungsskala entspricht grundsätzlich dem stärksten Schmerz S. Der Wert 0 der numerischen Schmerzbewertungsskala steht für die Abwesenheit von Schmerz.

Die Steuerung 14 im Laptop 16 erfasst verschiedene Daten speichert diese und wertet diese aus durch spezielle Algorithmen, wobei die Einzeldaten und Kombinationen aus verschiedenen Daten, umfassend sowohl Messdaten als auch patientenspezifische Daten verknüpft werden. Die Steuerung 14 wird bei den beiden hier vorgestellten Ausführungsbeispielen mit folgenden Daten versorgt:
- den eingestellten Abstand Δ der Kontakte 2, 3 der Klemmeinrichtung 1,
- die Zeit t, die benötigt wird, um eine Änderung der Gewebewiderstandskraft des Körpergewebes und damit dessen Lösung herbeizuführen,
- die Änderung der von der ersten Messeinrichtung 7 erfassten Werte der Klemmkraft FK in Korrelation zur Änderung der Gewebewiderstandskraft des Körpergewebes,
- den Maximalwert der Klemmkraft FK entsprechend dem Maximalwert der Gewebewiderstandskraft des Körpergewebes,
- Daten zur Messstelle, nämlich Körperteil, Anordnung und Abmessung des Hautabschnittes 4, 5,
- spezifische Daten zum Lebewesen, wie Schmerzempfindlichkeit, Geschlecht, Alter, Zustandsdaten betreffend Herzfrequenz, Blutdruck, Körpergröße, Körpergewicht, Fettanteil.

Bei beiden Ausführungsbeispielen der erfindungsgemäßen Vorrichtung ist zur Vermeidung von Hautquetschungen eine hier nicht dargestellte Sicherheitsstoppeinrichtung vorgesehen, die durch die Steuerung 14 und/oder durch das Lebewesen bzw. den menschlichen Patienten auslösbar ist, wenn ein bestimmter Abstand Δ der Kontaktauflagen 12, 13 der Klemmeinrichtung 1 unterschritten bzw. ein bestimmter Klemmkraftbetrag oder auch eine subjektive Schmerzgrenze überschritten wird.

Bei dem zweiten Ausführungsbeispiel der erfindungsgemäßen Vorrichtung ist als zusätzliche Sicherungsmaßnahme vorgesehen, dass sich die Kontakte 2, 3 der Klemmbacken 10, 11 zuzüglich der Kontaktauflagen 12, 13 nur soweit zusammenfahren lassen, dass ein Abstand Δ von 0,5 cm voneinander verbleibt.

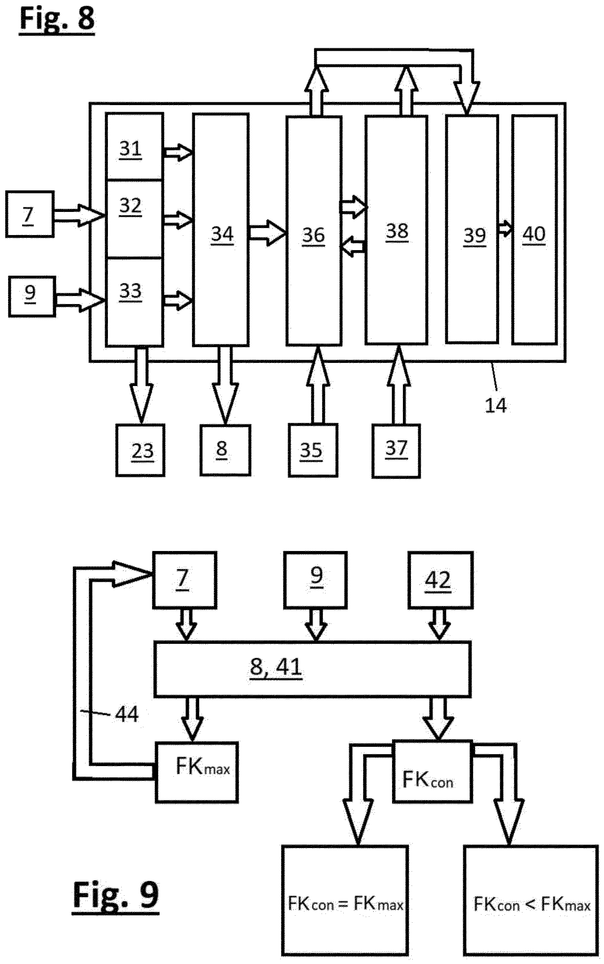

Bei beiden Ausführungsbeispielen enthält die Steuerung 14 einen in den Fig. 7 und 8 schematisch dargestellten Signalgeber 23, der verschiedene Signale im Zusammenhang mit den von der ersten Messeinrichtung 7 zugeführten Klemmkraftwerten abgibt, nämlich:
a) Ein optisches und ein akustisches Signal für den Fall, dass sich die Klemmkraft FK in Korrelation zur Gewebewiderstandskraft nach Erreichen eines Maximalwertes FKₘₐₓ, FK1ₘₐₓ , FK2ₘₐₓ vermindert.
b) Ein optisches und ein akustisches Signal für den Fall, dass die Klemmkraft FK in Korrelation zur Gewebewiderstandskraft einen konstanten Wert FK_{con} erreicht.

Bei beiden Ausführungsbeispielen umfasst die erfindungsgemäße Vorrichtung eine Halteeinrichtung 24 in Form eines Stativs, das bis an den Behandlungsplatz des Patienten gerollt werden kann. Die Klemmeinrichtung 1 ist an einem Seilzug 25 angeordnet, der ein Arretierungsmittel 26 umfasst, so dass die Höhe der Klemmeinrichtung 1 eingestellt werden kann. An der Klemmeinrichtung 1 sind Befestigungsmittel 27, 28 in Form von Ösen angeordnet, die mit dem Federzugring 29 am Ende des Seilzuges 25 zusammenwirken und die Kontakte 2, 3 in zwei verschieden Positionen zum Hautabschnitt 4, 5, verbringen können. In Fig. 2 ist die frontale Position in Bezug zum Patienten gezeigt. Wenn die Klemmeinrichtung 1 über das um 90° versetzte Befestigungsmittel 28 mit dem Federzugring 29 des Seilzuges verbunden ist, ergibt sich eine sagittale Position in Bezug zum Patienten. Zur Vorrichtung gehört ein fahrbarer Wagen 30, auf dem der Laptop 16 abgestellt ist. Gemäß Fig. 3 ist die Bedieneinheit 17 höhenverstellbar über nicht näher bezeichnete Befestigungsmittel am Stativ 24 befestigt und kann in den Sichtbereich des Patienten verbracht werden, so dass er auf der Anzeigeeinrichtung 8 der Bedieneinheit 17 die Darstellung der Klemmkraft FK, des Abstandes Δ zwischen den Kontakten 2, 3 und die Zeit t beobachten kann. Durch den verfahrbaren Wagen 30 geringerer Höhe gemäß Fig. 1 hat der Patient die Möglichkeit, die Anzeigeeinrichtung 8 des Laptops 16 zu betrachten und die dort abgebildeten Zustandsänderungen des Köpergewebes mitzuverfolgen.

Die Steuereinrichtung 14 ist mit Ihren Grund-Funktionen schematisch in Fig. 7 dargestellt. Die Steuereinrichtung 14 umfasst
- ein Zeitmessprogramm 31,
- ein Messwerterfassungsprogramm 32 für die von der zweiten Messeinrichtung 9 kommenden Messwerte betreffend den Abstand Δ zwischen den Kontakten 2, 3,
- ein Messwerterfassungsprogramm 33 für die von der ersten Messeinrichtung 7 kommenden Messwerte für die Klemmkraft FK in Korrelation zur Gewebewiderstandskraft,
- ein Rechenprogramm 34 zur Verknüpfung der Messwerte aus den Programmen 31, 32, 33 und zur Erstellung eines in Fig. 10 gezeigten Diagrammes 41 auf der Anzeigeeinrichtung 8.

Das Messwerterfassungsprogramm 33 für die von der ersten Messeinrichtung 7 kommenden Messwerte für die Klemmkraft FK in Korrelation zur Gewebewiderstandskraft enthält Erkennungsmittel für aufsteigende, konstante und absteigende Werte und ist mit dem Signalgeber 23 verbunden, der aktiviert wird, wenn die Klemmkraft wirkungslos geworden ist und ein neuer Schritt der Abstandsänderung der Kontakte 2, 3 im Hinblick auf die Behandlung des Patienten erforderlich ist.

Die Steuereinrichtung 14 umfasst gemäß einem weiteren, in Fig. 8 gezeigten Ausführungsbeispiel noch weitere Funktionen, die die Eingabe von patientenbezogenen Daten, deren Abgleich mit Daten in verschiedenen Datenbanken und eine Auswertung im Hinblick auf die anzuwendende Therapie erlauben.

Zum einen sind die Daten der Messstelle relevant, die vor der Behandlung erfasst und als Datenpaket 35 in eine Datenbank 36 eingespeist werden, welche eine Sammlung solcher messstellenbezogener Daten früherer Messungen an gesunden und pathologischen Körpergeweben und Behandlungen sowie früherer Diagramme und Therapieverläufe über die Zeit enthält. In die Datenbank 36 werden die aktuellen Messstellendaten, wie Körperteil, Größe und Platzierung des Hautabschnitts 4, 5, des Körpergewebes und auch das aktuelle, vom Rechenprogramm 34 ermittelte Diagramm zum Abgleich mit früheren Daten aus derselben Zuordnungsgruppe eingegeben.

Die vorgenannte Zuordnungsgruppe wird noch weiter präzisiert, indem noch Daten hinzutreten, die die Eigenschaften, den Zustand, Funktionen, die Beschaffenheit des Lebewesens betreffen. Dazu zählen Daten, wie Alter, Geschlecht, Größe, Gewicht, Körperfettanteil, Blutdruck, Herzfrequenz, die vor der Behandlung erfasst und als Datenpaket 37 in eine Datenbank 38 eingespeist werden. Die Datenbank 38 enthält eine Sammlung solcher Daten betreffend die körperliche Beschaffenheit von Lebewesen im Rahmen früherer Messungen und Behandlungen, die in Beziehung zu den Daten in der Datenbank 36 stehen. Das aktuelle Datenpaket 37 wird in der Steuerung 14 mit dem aktuellen Datenpaket 35 und dem aus dem Rechenprogramm 34 ermittelten aktuellen Diagramm in Beziehung gesetzt und schließlich mit der zutreffenden Gruppe früherer Daten der Datenbanken 36, 38 verglichen. Die aus dem Abgleich folgenden Resultate werden in eine Kontrolldatenbank 39 eingegeben, die Daten über anatomische Fakten enthält und von dort aus in ein Auswerteprogramm 40, das Therapievorschläge unterbreitet.

Zur Darstellung des Verfahrens werden die Figuren 9 und 10 erläutert. Die Fig. 9 zeigt ein Fließschema des Verfahrens, das die grundsätzlichen Verfahrensschritte umfasst. Bei der Fig. 10 wird der Zustand des Körpergewebes ermittelt, wobei ein aus dem Körpergewebe gebildeter Hautabschnitt 5 eines menschlichen Patienten zwischen zwei Kontakten 2, 3 eingeklemmt und in einem bestimmten Abstand Δ mit einer bestimmtem Klemmkraft FK beaufschlagt wird. Innerhalb von Einstell-Zeitintervallen t_{E} und Mess-Zeitintervallen t1, t2 wird die Klemmkraft FK gemessen und als Diagramm 41 auf der Anzeigeeinrichtung 8 dargestellt. Auch der Abstand Δ der Kontakte 2, 3 während der Mess-Zeitintervalle t1, t2 wird auf der Anzeigeeinrichtung 8 dargestellt. Ebenso ist aus Fig. 10 ersichtlich, dass der durch Befragung ermittelte Schmerz S eines menschlichen Patienten auf der Anzeigeeinrichtung 8 in Zuordnung zur maximalen Klemmkraft FK1ₘₐₓ und FK2ₘₐₓ sowie zur Klemmkraft FK am Ende t1_{E} und t2ε der beiden Zeitintervalle t1 und t2 angezeigt wird. Zuvor wird der subjektive Schmerzwert S manuell über die Tastatur in den Laptop 16 oder über das Bedienelement 22 in die Bedieneinheit 17 eingegeben.

In Fig. 9 ist gezeigt, dass die Messwerte der ersten Messeinrichtung 7 betreffend die Klemmkraft FK und der zweiten Messeinrichtung 9 betreffend den Abstand Δ zwischen den Kontakten 2, 3 bzw. der Kontaktauflagen 12, 13 sowie Zeitmesswerte 42 in einem Diagramm 41 in der Anzeigeeinrichtung 8 dargestellt werden. Je nach dem, ob ein Maximalwert FKₘₐₓ oder ein konstanter Wert FK_{con} der Klemmkraft FK in Korrelation zur Gebewiderstandskraft festgestellt wird, kommt es zu einer unterschiedlichen Auswertung. Wird ein Maximalwert der Klemmkraft FKₘₐₓ in Form einer Amplitude 43 festgestellt, so kommt es zu einer Neueinstellung der Klemmkraft FK gemäß Rückkopplung 44, was dann wieder zu neuen Messwerten der Messeinrichtungen 7 und 9 sowie neuen Zeitmesswerten 42 führt. Wird dagegen ein konstanter Klemmkraftwert FK_{con} festgestellt, so wird unterschieden, ob FK_{con} = FKₘₐₓ oder FK_{con} < FKₘₐₓ. Ist FK_{con} = FKₘₐₓ wird Therapieresistenz auf höchstem Schmerzniveau festgestellt und die Behandlung ist zu stoppen. Eine neue Therapie ist zu überlegen. Ist FK_{con} < FKₘₐₓ wird Therapieresistenz auf einem etwas erträglicherem Schmerzniveau und damit eine vergrößerte Belastungstoleranz festgestellt und die Behandlung ist zu stoppen. Entweder handelt es sich um das bestmöglich zu erreichende Ergebnis im Hinblick auf die Lösung / Entspannung / Entstauung des behandelten Körpergewebes oder es ist eine neue Therapie zu überlegen.

Nachfolgend wird im Zusammenhang mit Fig. 10 eine Anwendung der erfindungsgemäßen Vorrichtung und des erfindungsgemäßen Verfahrens auf eine zwei Schritte umfassende Behandlung einer Messstelle, betreffend eine Impfnarbe am rechten Oberarm beschrieben. Die Messstelle ist ein Hautabschnitt 5.

Das Diagramm 41 gemäß Fig. 10 zeigt den Klemmkraftverlauf in Korrelation zur Änderung der Gewebewiderstandskraft des behandelten Hautabschnittes 5 in zwei Mess-Zeitintervallen t1 und t2, die die beiden Behandlungsschritte bei konstantem Abstand Δ zwischen den Kontakten 2, 3 repräsentieren. Außerdem enthält das Diagramm 41 Informationen zum Klemmkraftverlauf innerhalb von Einstell-Zeitintervallen t_{E}, die den Mess-Zeitintervallen t1, t2 vorgeschaltet sind.

### Messen und Auswerten während des ersten Behandlungsschrittes:

Im Einstell-Zeitintervall t_{E} in der Zeit t = 0 s bis t = 30 s wird die bewegliche Klemmbacke 11 zur festen Klemmbacke 10 bewegt und es wird eine Feineinstellung vorgenommen. In diesem Einstell-Zeitintervall t_{E} kommt es zum Anstieg der Klemmkraft FK.

Bei t = 30 s wird der Maximalwertes FK1ₘₐₓ. = 2350 N unter Ausbildung einer Amplitude 43 erreicht. Die gerade noch aushaltbare subjektive Schmerzwahrnehmung beträgt S = 8 und wird manuell in die Datenverarbeitungseinrichtung 16 eingegeben und auf der Anzeigeeinrichtung 8 angezeigt - und zwar auf der Hilfslinie, die zum Wert der Klemmkraft FK1ₘₐₓ = 2350 N zum Zeitpunkt t = 30 s gehört. Die Arretierung der beweglichen Klemmbacke 11 im Abstand Δ von 20 mm zwischen den Kontakten 2, 3 bzw. zwischen den Kontaktauflagen 12, 13 wird zum Zeitpunkt t = 30 s durchgeführt. Der Hautabschnitt 5 ist zwischen den Kontaktauflagen 12, 13 wirksam eingeklemmt. Der Wert t = 30 s definiert den Anfang t1_{A} des ersten Mess-Zeitintervalls t1 und der Wert t = 60 s definiert das Ende t1_{E} des ersten Mess-Zeitintervalls t1, innerhalb dessen die Klemmkraftbeaufschlagung gemessen, erfasst und ausgewertet wird.

Im ersten Mess-Zeitintervall t1 von insgesamt 30 s, nämlich von t1_{A} = 30 s bis t1_{E} = 60 s, bleibt der Abstand Δ zwischen den Kontakten 2, 3 gleich und die Klemmkraft FK verringert sich bis zu einem Klemmkraftwert von 450 N bei t1_{E} = 60 s am Ende des ersten Zeitintervalls t1, wobei die subjektive Schmerzwahrnehmung S = 2 beträgt.

Nach Erreichen des Wertes 450 N der Klemmkraft FK signalisiert der Signalgeber 23, dass die Klemmkraft FK soweit nachgelassen hat, dass der Abstand Δ von 20 mm nunmehr effektlos geworden ist, da die Lösung des Körpergewebes eingesetzt hat. Somit endet der erste Behandlungsschritt nach 60 s Gesamtzeit und 30 s des ersten Zeitintervalls während des festgelegten Abstand Δ von 20 mm. Das Körpergewebe der Hautfalte 5 ist gelöst und die Gewebewiderstandskraft entspricht dem Wert der Klemmkraft FK im am Ende t1_{E} des ersten Zeitintervalls t1.

### Messen und Auswerten während des zweiten Behandlungsschrittes:

Im Einstell-Zeitintervall t_{E} in der Zeit t = 60 s bis t = 80 s wird die bewegliche Klemmbacke 11 erneut zur festen Klemmbacke 10 bewegt, und zwar beginnend auf dem Niveau des Wertes der Klemmkraft FK in Höhe von 450 N und es wird erneut eine Feineinstellung vorgenommen. In diesem Einstell-Zeitintervall t_{E} kommt es erneut zum Anstieg der Klemmkraft FK. Der Abstand Δ der Kontakte 2, 3 bzw. deren Kontaktauflagen 12, 13 verringert sich dabei um 3 mm.

Bei t = 80 s wird der Maximalwertes FK2ₘₐₓ. = 3445 N unter Ausbildung einer Amplitude 43 erreicht. Die extremste beim Patienten abgefragte subjektive Schmerzwahrnehmung beträgt S = 6 und wird manuell in die Datenverarbeitungseinrichtung 16 eingegeben und auf der Anzeigeeinrichtung 8 angezeigt - und zwar auf der Hilfslinie, die zum Wert der Klemmkraft FK2ₘₐₓ = 3445 N zum Zeitpunkt t = 80 s gehört. Die Arretierung der beweglichen Klemmbacke 11 im Abstand Δ von 17 mm zwischen den Kontakten 2, 3 bzw. zwischen den Kontaktauflagen 12, 13 wird zum Zeitpunkt t = 80 s durchgeführt. Der Hautabschnitt 5 ist zwischen den Kontaktauflagen 12, 13 wirksam eingeklemmt. Der Wert t = 80 s definiert den Anfang t2_{A} des zweiten Mess-Zeitintervalls t2 und der Wert t = 110 s definiert das Ende t2ε des zweiten Mess-Zeitintervalls t2, innerhalb dessen die Klemmkraftbeaufschlagung gemessen, erfasst und ausgewertet wird.

Im zweiten Mess-Zeitintervall t2 von insgesamt 30 s, nämlich von t2_{A} = 80 s bis t2ε = 110 s, bleibt der Abstand Δ zwischen den Kontakten 2, 3 gleich und die Klemmkraft FK verringert sich wieder bis zu einem Klemmkraftwert von 450 N bei t2ε = 110 s am Ende des zweiten Mess-Zeitintervalls t2, wobei die subjektive Schmerzwahrnehmung S = 2 beträgt.

Nach Erreichen des Wertes 450 N der Klemmkraft FK signalisiert der Signalgeber 23, dass die Klemmkraft FK soweit nachgelassen hat, dass der Abstand Δ von 17 mm nunmehr effektlos geworden ist, da die Lösung des Körpergewebes eingesetzt hat. Somit endet der zweite Behandlungsschritt nach 110 s Gesamtzeit und 30 s des zweiten Mess-Zeitintervalls t2 während des festgelegten Abstandes Δ von 17 mm.

Der Vergleich der Werte des Schmerzes S = 8 bei einer Klemmkraft FK1ₘₐₓ von 2350 N im ersten Behandlungsschritt und S = 6 bei einer Klemmkraft FK2ₘₐₓ von 3445 N im Zweiten Behandlungsschritt zeigt, dass nach dem ersten Behandlungsschritt eine höhere Belastungstoleranz bei geringerer Schmerzwahrnehmung erreicht wurde. Außerdem zeigt der Wert des Schmerzes S = 2 bei einer Klemmkraft von FK = 450 N, dass bei beiden Behandlungsschritten, trotz verringertem Abstand Δ die Wirkungslosigkeit der Klemmeinrichtung 1 bei demselben Klemmkraftwert eintritt.

Diese Auswertung ermöglicht die Dokumentation der Zustandsänderung des Narbengewebes der Impfnarbe des rechten Oberarms. Faszienverklebungen haben sich nachweisbar und darstellbar durch das Nachlassen der die Gewebewiderstandskraft des Hautabschnittes 5 spiegelnden Klemmkraft FK gelöst, haben eine höhere Belastungstoleranz und können Ihre Funktion wieder besser wahrnehmen.

Bei beiden Schritten des Verfahrens ist die Arretierung der beweglichen Klemmbacke 11 beim Erreichen der Amplituden 43 bzw. den Maximalwerten der Klemmkräfte FK1ₘₐₓ und FK2ₘₐₓ erfolgt, was eine besonders hohe Effizienz hinsichtlich der Anwendung der Vorrichtung und des Verfahrens gemäß der Erfindung mit sich bringt.

Hinsichtlich weiterer, in den Figuren nicht gezeigter Merkmale wird auf den allgemeinen Teil der Beschreibung verwiesen.

Abschließend sei darauf hingewiesen, dass die Lehre nicht auf die voranstehend erörterten Ausführungsbeispiele eingeschränkt ist. Vielmehr kann das Verfahren durch Symmetriemessungen und Vergleich mit Daten aus Datenbanken, Eingabe weiterer Messdaten, erheblich erweitert werden. Die Vorrichtung kann anders ausgestaltet Kontakte aufweisen und die Steuerung kann weitere Messdaten aufnehmen und auswerten.

Die Erfindung ist in den anhängenden Ansprüchen definiert.

**Bezugszeichenliste**

| | | | | |
|---|---|---|---|---|
| 1 | Klemmeinrichtung | | 30 | Wagen |
| 2 | Kontakt | | 31 | Zeitmessprogramm |
| 3 | Kontakt | | 32 | Abstandmesswerterfassungsprogramm |
| 4 | Hautabschnitt | | | |
| 5 | Hautabschnitt | | 33 | Klemmkraftwerteerfassungsprogramm |
| 6 | Einstelleinrichtung | | | |
| 7 | Erste Messeinrichtung | | 34 | Rechenprogramm |
| 8 | Anzeigeeinrichtung | | 35 | Datenpaket |
| 9 | Zweite Messeinrichtung | | 36 | Datenbank |
| 10 | Bewegliche Klemmbacke | | 37 | Datenpaket |
| 11 | Feste Klemmbacke | | 38 | Datenbank |
| 12 | Kontaktauflage | | 39 | Kontrolldatenbank |
| 13 | Kontaktauflage | | 40 | Auswerteprogramm |
| 14 | Steuerung | | 41 | Diagramm |
| 15a | Elektrische Leitung | | 42 | Zeitmesswerte |
| 15b | Messwerteaufbereitung | | 43 | Amplitude |
| 15c | Elektrische Leitung | | 44 | Rückkopplung |
| 16 | Datenverarbeitungsgerät, Laptop | | | |
| 17 | Datenverarbeitungsgerät, Bedieneinheit | | | |
| 18 | Bedienelement | | | |
| 19 | Bedienelement | | | |
| 20 | Bedienelement | | | |
| 21 | Bedienelement | | | |
| 22 | Bedienelement | | | |
| 23 | Signalgeber | | | |
| 24 | Halteeinrichtung, Stativ | | | |
| 25 | Seilzug | | | |
| 26 | Arretierungsmittel | | FK | Klemmkraft |
| 27 | Befestigungsmittel | | t | Zeit |
| 28 | Befestigungsmittel | | Δ | Abstand |
| 29 | Federzugring | | S | Schmerz |
| A, B, C, D | | Pfeile | | |
| FKₘₐₓ / FK1ₘₐₓ / FK2ₘₐₓ | | Maximale Klemmkraft | | |
| FK_{con} | | Konstante Klemmkraft | | |
| t_{E} | | Einstell-Zeitintervall | | |
| t1 - t1_{A} bis t1_{E} | | Erstes Mess-Zeitintervall - t1 Anfang bis t1 Ende | | |
| t2 - t2_{A} bis t2_{E} | | Zweites Mess-Zeitintervall - t2 Anfang bis t2 Ende | | |

## Patentansprüche

1. Vorrichtung zur Einwirkung auf das Körpergewebe eines Lebewesens mit einer Klemmeinrichtung (1) mit voneinander beabstandeten Kontakten (2, 3) zum Einklemmen eines aus Körpergewebe gebildeten Hautabschnitts (4, 5) des Lebewesens, mit einer Einstelleinrichtung (6) zur Einstellung der auf das Körpergewebe aufgebrachten Klemmkraft (FK), mit einer ersten Messeinrichtung (7) zur Ermittlung der auf den Hautabschnitt (4, 5) aufgebrachten Klemmkraft (FK), mit einer Anzeigeeinrichtung (8) zur Darstellung der Messwerte der ersten Messeinrichtung (7), und mit einer zweiten Messeinrichtung (9) zur Erfassung des Abstandes (Δ) zwischen den Kontakten (2, 3),
**dadurch gekennzeichnet, dass** die Vorrichtung so eingerichtet ist, dass auf der Anzeigeeinrichtung (8) die Veränderung der Klemmkraft (FK) in Korrelation zur Veränderung der Gewebewiderstandskraft des Hautabschnittes (4, 5) während eines Mess-Zeitintervalls (t1, t2) bei konstantem Abstand (Δ) der Kontakte (2, 3) darstellbar ist.

2. Vorrichtung nach Anspruch 1, **dadurch gekennzeichnet, dass** die Klemmeinrichtung (1) eine feststehende Klemmbacke (10) und eine bewegliche Klemmbacke (11) aufweist, an denen sich die Kontakte (2, 3), vorzugsweise mit Kontaktauflagen (12, 13) versehen, befinden, und dass die erste Messeinrichtung (7) der feststehenden Klemmbacke (10) und die zweite Messeinrichtung (9) der beweglichen Klemmbacke (11) zugeordnet sind.

3. Vorrichtung nach Anspruch 1 oder 2, **dadurch gekennzeichnet, dass** eine vorzugsweise Eingabemittel, Auswahlmittel und Speichermittel umfassende Steuerung (14) vorgesehen ist, die mit den Messeinrichtungen (7, 9) und der Anzeigeeinrichtung (8) zusammenwirkt, wobei zumindest ein Datentransfer stattfindet, der elektrisch und/oder über Sender-Empfänger-Einheiten berührungslos stattfindet.

4. Vorrichtung nach Anspruch 3, **dadurch gekennzeichnet, dass** die Steuerung (14) verschiedene Daten erfasst und verwertet sowie speichert, insbesondere
• den eingestellten Abstand (Δ) der Kontakte (2, 3) der Klemmeinrichtung (1),
• die Zeit (t), die benötigt wird, um eine Änderung der Gewebewiderstandskraft des Körpergewebes und damit dessen Lösung herbeizuführen,
• die von der ersten Messeinrichtung (7) erfassten Werte der Klemmkraft (FK) in Korrelation zur Änderung der Gewebewiderstandskraft des Körpergewebes,
• den Maximalwert der Klemmkraft (FK) entsprechend dem Maximalwert der Gewebewiderstandskraft des Körpergewebes,
• vorzugsweise Daten zur Messstelle, wie Körperteil, Anordnung und Abmessung des Hautabschnittes (4, 5),
• vorzugsweise spezifische Daten zum Lebewesen, wie bspw. Schmerzempfindlichkeit, Geschlecht, Alter, Zustandsdaten betreffend Herzfrequenz, Blutdruck, Körpergröße, Körpergewicht, Fettanteil.

5. Vorrichtung nach Anspruch 3 oder 4, **dadurch gekennzeichnet, dass** eine Sicherheitstoppeinrichtung vorgesehen ist, die durch die Steuerung (14) und/oder durch das Lebewesen auslösbar ist, wenn ein bestimmter Abstand (Δ) der Kontakte (2, 3) der Klemmeinrichtung (1) unterschritten bzw. wenn ein bestimmter Wert der Klemmkraft (FK) überschritten wird.

6. Vorrichtung nach einem der Ansprüche 1 bis 5, **dadurch gekennzeichnet, dass** die erste Messeinrichtung (7) zur Erfassung der Klemmkraft (FK) oder ggf. ein Klemmkraftwerteerfassungsprogramm (33) der Steuerung (14) einen Signalgeber (23) umfasst, welcher verschiedene Zustände signalisiert, insbesondere:
a) Verminderung der Klemmkraft (FK) in Korrelation zur Gewebewiderstandskraft nach Erreichen eines Maximalwertes (Amplitude (43)
b) Gleichbleiben der Klemmkraft (FK) in Korrelation zur Gewebewiderstandskraft, insbesondere nach Erreichen eines Maximalwertes.

7. Vorrichtung nach einem der Ansprüche 1 bis 6, **dadurch gekennzeichnet, dass** eine Halteeinrichtung (24), insbesondere in Form eines Stativs (24), vorgesehen ist, an der die Klemmeinrichtung (1) angeordnet ist, wobei die Halteeinrichtung (24) und die Klemmeinrichtung (1) über Befestigungsmittel (27, 28) miteinander lösbar verbindbar sind, die die Anordnung der Klemmeinrichtung (1) in verschiedenen Positionen in Bezug zum Lebewesen erlauben.

8. Vorrichtung nach einem der Ansprüche 1 bis 7 **dadurch gekennzeichnet, dass** eine Wärmebildkamera vorgesehen ist, um Stauungen, Verklebungen, Zustandsänderungen des Körpergewebes im Hautabschnitt (4, 5) darzustellen.

9. Verfahren zur Messung und Auswertung eines Körpergewebezustandes, unter Einsatz einer Vorrichtung gemäß einem der Ansprüche 1 bis 8, wobei ein aus Körpergewebe gebildeter Hautabschnitt eines Lebewesens zwischen zwei Kontakten eingeklemmt und mit einer einstellbaren Klemmkraft beaufschlagt wird, wobei die Klemmkraft und der Abstand zwischen den Kontakten gemessen wird und wobei die gemessenen Klemmkraftwerte auf einer Anzeigeeinrichtung dargestellt werden, wobei das Verfahren weiter umfasst,
• dass der Abstand der Kontakte innerhalb eines Einstell-Zeitintervalls auf einen konstanten Wert eingestellt wird, wobei der Hautabschnitt zwischen den Kontakten eingeklemmt wird, und es zum Anstieg der Klemmkraft kommt,
• dass der konstante Wert des Abstandes der Kontakte und das Ende des Einstell-Zeitintervalls durch die gerade noch aushaltbare subjektive Schmerzwahrnehmung vorgegeben werden und dann eine Arretierung der Kontakte erfolgt,
• dass am Ende des Einstell-Zeitintervalls und nach der Arretierung der Kontakte im konstanten Abstand das Mess-Zeitintervall einsetzt und
• dass während des Mess-Zeitintervalls die Änderung der Klemmkraft in Korrelation zur Gewebewiderstandskraft gemessen und auf der Anzeigeeinrichtung dargestellt wird.

10. Verfahren nach Anspruch 9, **dadurch gekennzeichnet, dass** im Mess-Zeitintervall ein Maximalwert der Klemmkraft in Korrelation zur Gewebewiderstandskraft des Körpergewebes ermittelt wird und dass der Maximalwert dahingehend ausgewertet wird, ob er eine Amplitude umfasst oder einen konstanten Verlauf aufweist.

11. Verfahren nach Anspruch 10, **dadurch gekennzeichnet, dass** bei Feststellung eines Maximalwertes der Klemmkraft in Korrelation zur Gewebewiderstandskraft, umfassend eine Amplitude, eine Neueinstellung der Klemmkraft durch Verringerung des Abstandes der Kontakte erfolgt und dass bei Feststellung eines konstanten Maximalwertes der Gewebewiderstandskraft die Klemmkraftbeaufschlagung gestoppt wird.

12. Verfahren nach Anspruch 11, **dadurch gekennzeichnet,**
a) **dass** im Fall der Feststellung eines Maximalwertes der Klemmkraft in Korrelation zur Gewebewiderstandskraft in Form einer Amplitude Informationen zum Schmerzaufbau und zum Fortschreiten der Lösung von Körpergewebe sowie zum Schmerzabbau nach der Lösung des Körpergewebes gewonnen werden und
b) **dass** im Fall der Feststellung eines konstanten Maximalwertes der Klemmkraft in Korrelation zur Gewebewiderstandskraft Informationen zur Therapieresistenz gewonnen werden.

13. Verfahren nach einem der Ansprüche 9 bis 12, **dadurch gekennzeichnet, dass** mindestens zwei aus Körpergewebe symmetrischer Körperteile gebildete Hautabschnitte zwischen den Kontakten eingeklemmt und die gemessenen Werte der Klemmkraft in Korrelation zur Gewebewiderstandskraft bei konstantem Abstand der Kontakte einer Klemmeinrichtung miteinander verglichen werden.

14. Verfahren nach einem der Ansprüche 9 bis 13, **dadurch gekennzeichnet, dass** parallel zur Messung und Anzeige der Klemmkraft in Korrelation zur Gewebewiderstandskraft eine Schmerzabfrage eines menschlichen Lebewesens durchgeführt wird, wobei die Schmerzangaben des menschlichen Lebewesens zu dessen Schutz vor unzutreffender Wahrnehmung vorzugsweise kontrolliert werden, indem vor und während der Krafteinwirkung ermittelte Werte betreffend den Blutdruck und / oder die Herzfrequenz miteinander verglichen und erforderlichenfalls ein Abschalten der Vorrichtung herbeigeführt wird.

15. Verfahren nach einem der Ansprüche 9 bis 14, **dadurch gekennzeichnet, dass** vor, ggf. während und nach der Krafteinwirkung ermittelte Temperaturwerte im Bereich des Hautabschnitts miteinander verglichen werden und/oder dass vor und nach der Krafteinwirkung ermittelte Seratoninwerte miteinander verglichen werden.

16. Verfahren nach einem der Ansprüche 9 bis 15, **dadurch gekennzeichnet, dass** zum Zweck einer Verallgemeinerungsauswertung Datensammlungen zu Behandlungszykluszeiten, Wiederholungen der Krafteinwirkungen ähnlicher Hautabschnitte von Lebewesen mit ähnlichen Eigenschaften und ähnlichem pathologischen Körpergewebe angelegt und zur Erstellung von Algorithmen verwendet werden.

## Claims

1. Device for acting on the body tissue of a living being with a clamping device (1) with contacts (2, 3) at a distance from each other for clamping a section of skin (4, 5) of the living being formed of body tissue, with an adjusting device (6) for adjusting the clamping force (FK) applied to the body tissue, with a first measuring device (7) for determining the clamping force (FK) applied to the section of skin (4, 5), with a display device (8) for showing the measurements of the first measuring device (7), and with a second measuring device (9) for recording the distance (Δ) between the contacts (2, 3),
**characterised in that** the device is configured in such a way that the change in the clamping force (FK) in correlation to the change in the tissue resistance of the section of skin (4, 5) during a measuring time interval (t1, t2) with a constant distance (Δ) between the contacts (2, 3) can be shown on the display device (8) .

2. Device according to claim 1, **characterised in that** the clamping device (1) comprises a fixed clamping jaw (10) and a moveable clamping jaw (11) on which are located the contacts (2, 3), preferably provided with contact supports (12, 13) and **in that** the first measuring device (7) is assigned to the fixed clamping jaw (10) and the second measuring device (9) to the moveable clamping jaw (11).

3. Device according to claim 1 or 2, **characterised in that** a control device (14), preferably comprising input means, selection means and memory means, is provided, which interacts with the measuring devices (7, 9) and the display device (8), wherein at least one data transfer takes place, which takes place electrically and/or via transmitter-receive units in a contactless manner.

4. Device according to claim 3, **characterised in that** the control device (14) records, processes and stores various data, more particularly
• the set distance (Δ) between the contacts (2, 3) of the clamping device (1),
• the time (t) required to bring about a change in the tissue resistance of the body tissue and therefore is separation,
• the values of the clamping force (FK) recorded by the first measuring device (7) in correlation to the change in the tissue resistance of the body tissue,
• the maximum value of the clamping force (FK) corresponding to the maximum value of the tissue resistance of the body tissue,
• preferably data about the measuring point, such as part of the body, arrangement and dimension of the section skin (4, 5),
• preferably specific data about the living being, such as pain sensitivity, gender, age, condition-related data such as heart rate, blood pressure, height, weight, fat proportion.

5. Device according to claim 3 of 4, **characterised in that** a safety stop device is provided which can be triggered by the control device (14) and/or by the living being if a certain distance (Δ) between the contacts (2, 3) of the clamping device (1) is not reached or if a certain clamping force (FK) value is exceeded.

6. Device according to any one of claims 1 to 5, **characterised in that** the first measuring device (7) for recording the clamping force (FK) or, possibly, a clamping force value recording programme (33) of the control device (14) comprises a signal emitter (23), which signals various states, more particularly:
a) reduction in the clamping force (FK) in correlation to the tissue resistance force after reaching a maximum value (amplitude) (43)
b) the clamping force (FK) staying the same in correlation to the tissue resistance force, in particular after reaching a maximum value.

7. Device according to any one of claims 1 to 6, **characterised in that** a holding device (24), more particularly in the form of a stand (24) is provided, on which the clamping device (1) is arranged, wherein the holding device (24) and the clamping device (1) are connected to each other in a detachable manner by means of fastening means (27, 28), which allow the arrangement of the clamping device (1) in various positions in relation to the living being.

8. Clamping device according to any one of claims 1 to 7, **characterised in that** a heat imaging camera is provided in order to show blockages, adhesions, changes in the condition of the body tissue in the section of skin (4, 5) .

9. Method of measuring and evaluating a body tissue state, using a device according to any one of claims 1 to 8, wherein a section of skin of a living being formed of body tissue is clamped between two contacts and subjected to an adjustable clamping force, wherein the clamping force and the distance between the contacts is measured, and wherein the measured clamping force values are shown on a display device, wherein the method further includes the fact
• that the distance between the contacts is adjusted to a constant value within an adjusting time interval, wherein the section of skin is clamped between the contacts and an increase in the clamping force occurs,
• that the constant value of the distance between the contacts, and the end of the adjusting time interval are predefined by the still just bearable subjective pain perception and then stopping of the contacts occurs,
• that at the end of the adjusting time interval and after stopping of the contacts at a constant distance, the measuring time interval starts and
• that during the measuring time interval, the changes in clamping force in correlation to the tissue resistance force is measured and shown on the display device.

10. Method according to claim 9, **characterised in that** in the measuring time interval, a maximum value of the clamping force in correlation to the tissue resistance force of the body tissue is determined and **in that** the maximum value is evaluated as to whether it includes an amplitude or has a constant course.

11. Method according to claim 10, **characterised in that** on determining a maximum value of the clamping force in correlation to the tissue resistance force, comprising an amplitude, a new adjustment of the clamping force takes place through reducing the distance between the contacts and **in that** on determining a constant maximum value of the tissue resistance force, the exertion of the clamping force is stopped.

12. Method according to claim 11, **characterised in that**
a) in the event of determining a maximum value of the clamping force in correlation to the tissue resistance force in the form of an amplitude, information about the build up of pain and progress of the separation of the body tissue and about the decrease in pain after separation of the body tissue is obtained and
b) that in the case of determining a constant maximum value of the clamping force in correlation to the tissue resistance force, information on the therapy resistance is obtained.

13. Method according to any one of claims 9 to 12, **characterised in that** at least two sections of skin formed of body tissue of symmetrical parts of the body are clamped between the contacts and the measured values of the clamping force in correlation to the tissue resistance force at a constant distance between the contacts of clamping device are compared with each other.

14. Method according to any one of claims 9 to 13, **characterised in that** in parallel to the measuring and display of the clamping force in correlation to the tissue resistance face, a pain survey of a human living being is carried out, wherein to protect against inapplicable perception, the pain statements of the human living being are preferably monitored **in that** before and during the exerted force, determined values relating to blood pressure and/or heart rate are compared with each other and if necessary the device is switched off.

15. Method according to any one of claims 9 to 14, **characterised in that** before, possibly during, and after the exertion of force, determined temperature values in the area of the section of skin are compared with each other and/or **in that** before and after the exertion of force, determined serotonin values are compared with each other.

16. Method according to any one of claims 9 to 15, **characterised in that** for the purpose of a generalisation evaluation, data collections on treatment cycle times, repetitions of the force exertions of similar sections of skin of living being with similar properties and similar pathological body tissue are created and used for the establishment of algorithms.

## Revendications

1. Dispositif, destiné à agir sur le tissu corporel d'un être vivant, pourvu d'un système de serrage (1) doté de contacts (2, 3) écartés les uns des autres, pour enserrer une partie cutanée (4, 5) de l'être vivant constituée de tissu corporel, pourvue d'un système de réglage (6), destiné à régler la force de serrage (FK) appliquée sur le tissu corporel, avec un premier système de réglage (7) pour déterminer la force de serrage (FK) appliquée sur la partie cutanée (4, 5), avec un système d'affichage (8) pour représenter les valeurs de mesure du premier système de réglage (7) et avec un deuxième système de réglage (9), pour détecter l'écart (Δ) entre les contacts (2, 3),
**caractérisé en ce que** le dispositif est configuré de telle sorte que sur le système d'affichage (8), la variation de la force de serrage (FK) en corrélation avec la variation de la force de résistance du tissu de la partie cutanée (4, 5) pendant un intervalle de temps de mesure (t1, t2) avec un écart constant (Δ) des contacts (2, 3) puisse être représentée.

2. Dispositif selon la revendication 1, **caractérisé en ce que** le système de serrage (1) comporte une mâchoire de serrage (10) stationnaire et une mâchoire de serrage (11) mobile, sur lesquelles se trouvent les contacts (2, 3), munis de préférence de supports de contact (12, 13) et **en ce que** le premier système de réglage (7) est associé à la mâchoire de serrage (10) stationnaire et le deuxième système de réglage (9) est affecté à la mâchoire de serrage (11) mobile.

3. Dispositif selon la revendication 1 ou 2, **caractérisé en ce qu'**il est prévu un système de commande (14) comprenant de préférence des moyens de saisie, des moyens de sélection et des moyens de mémorisation, qui interagit avec les systèmes de mesure (7, 9) et avec le système d'affichage (8), au moins un transfert de données étant effectué, qui est effectué sans contact, par voie électrique et / ou par l'intermédiaire d'unités émetteur / récepteur.

4. Dispositif selon la revendication 3, **caractérisé en ce que** le système de commande (14) détecte et évalue et mémorise différentes données, notamment
• l'écart (Δ) réglé entre les contacts (2, 3) du système de serrage (1),
• le temps (t), qui est requis pour provoquer une variation de la force de résistance de tissu du tissu corporel et ainsi son détachement,
• les valeurs détectées par le premier système de réglage (7) pour la force de serrage (FK) en corrélation avec la force de résistance de tissu du tissu corporel,
• la valeur maximum de la force de serrage (FK), correspondant à la valeur maximum de la force de résistance de tissu du tissu corporel,
• de préférence des données concernant le point de mesure, telle que la partie corporelle, l'emplacement et les dimensions de la partie cutanée (4, 5),
• de préférence des données spécifiques concernant l'être vivant, telles que par exemple sa sensibilité à la douleur, son sexe, sont âge, ses données d'état concernant sa fréquence cardiaque, sa pression artérielle, sa taille, son poids, sa masse graisseuse.

5. Dispositif selon la revendication 3 ou 4, **caractérisé en ce qu'**il est prévu un système d'arrêt de sécurité, qui est déclenchable par le système de commande (14) et / ou par l'être vivant, lorsqu'un écart (Δ) déterminé des contacts (2, 3) du système de serrage (1) n'est pas atteint ou si une valeur déterminée de la force de serrage (FK) est dépassée.

6. Dispositif selon l'une quelconque des revendications 1 à 5, **caractérisé en ce que** le premier système de réglage (7) destiné à détecter la force de serrage (FK) ou le cas échéant un programme de détection (33) des valeurs de la force de serrage du système de commande (14), comprend un générateur de signaux (23), lequel signale différents états, notamment :
a) une réduction de la force de serrage (FK), en corrélation avec la force de résistance de tissu, par l'atteinte d'une valeur maximum (amplitude (43)
b) une constance de la force de serrage (FK), en corrélation avec la force de résistance de tissu, notamment après atteinte d'une valeur maximum.

7. Dispositif selon l'une quelconque des revendications 1 à 6, **caractérisé en ce qu'**il est prévu un système de maintien (24), notamment sous la forme d'un trépied (24), sur lequel est placé le système de serrage (1), le système de maintien (24) et le système de serrage (1) étant susceptibles d'être assemblés l'un avec l'autre de manière amovible par l'intermédiaire de moyens de fixation (27, 28) qui permettent le placement du système de serrage (1) dans différentes positions par rapport à l'être vivant.

8. Dispositif selon l'une quelconque des revendications 1 à 7 **caractérisé en ce qu'**il est prévu une caméra thermographique, destinée à représenter des congestions, des adhérences, des variations d'état du tissu corporel dans la partie cutanée (4, 5) .

9. Procédé, destiné à mesurer et à évaluer un état d'un tissu corporel,
en utilisant un dispositif selon l'une quelconque des revendications 1 à 8, une partie cutanée d'un être vivant constituée de tissu corporel étant enserrée entre deux contacts et soumise à une force de serrage, la force de serrage et l'écart entre les contacts étant mesurés et les valeurs mesurées de la force de serrage étant représentées sur un système d'affichage, le procédé comprenant par ailleurs,
• que l'écart entre les contacts est réglé à une valeur constante à l'intérieur d'un intervalle de temps de réglage, la partie cutanée étant insérée entre les contacts et une augmentation de la force de serrage étant observée,
• que la valeur constante de l'écart entre les contacts et la fin de l'intervalle de temps de réglage sont prédéfinis par la perception de la douleur juste encore supportable et en ce qu'un blocage des contacts a alors lieu,
• en ce qu'à la fin de l'intervalle de temps de réglage et après le blocage des contacts, l'intervalle de temps de mesure intervient avec un écart constant et
• en ce que pendant l'intervalle de temps de mesure, la variation de la force de serrage est mesurée en corrélation avec la force de résistance de tissu et représentée sur le système d'affichage.

10. Procédé selon la revendication 9, **caractérisé en ce que** dans l'intervalle de temps de mesure, une valeur maximum de la force de serrage en corrélation avec la force de résistance de tissu du tissu corporel est déterminée et **en ce que** la valeur maximum est évaluée pour savoir si elle comprend une amplitude ou si elle présente une courbe constante.

11. Procédé selon la revendication 10, **caractérisé en ce que** si une valeur maximum de la force de serrage en corrélation avec la force de résistance de tissu, comprenant une amplitude est constatée, un nouveau réglage de la force de serrage est effectué par réduction de l'écart entre les contacts et **en ce que** si une valeur maximum constante de la force de résistance de tissu est constatée, la soumission à la force de serrage est arrêtée.

12. Procédé selon la revendication 11, **caractérisé,**
a) **en ce que** dans la cas d'une constatation d'une valeur maximum de la force de serrage en corrélation avec la force de résistance de tissu sous la forme d'une amplitude, des informations concernant le développement de la douleur et la poursuite du détachement de tissu corporel, ainsi que le développement de la douleur après la dissolution du tissu corporel sont acquises et
b) **en ce que** dans la cas d'une constatation d'une valeur maximum constante de la force de serrage en corrélation avec la force de résistance de tissu, des informations sont acquises au niveau de la résistance à la thérapie.

13. Procédé selon l'une quelconque des revendications 9 à 12, **caractérisé en ce qu'**au moins deux parties cutanées constituées de tissu corporel de parties corporelles symétriques sont enserrées entre les contacts et **en ce que** les valeurs mesurées de la force de serrage en corrélation avec la force de résistance de tissu avec un écart constant des contacts d'un système de serrage sont comparées les unes avec les autres.

14. Procédé selon l'une quelconque des revendications 9 à 13, **caractérisé en ce que** parallèlement à la mesure et à l'affichage de la force de serrage en corrélation avec la force de résistance de tissu, il est procédé à un questionnement sur la douleur d'un être vivant humain, les indications concernant la douleur de l'être vivant humain étant de préférence contrôlées pour le protéger d'une perception incorrecte **en ce que** des valeurs déterminées avant et pendant l'action de la force concernant la pression sanguine et / ou la fréquence cardiaque sont comparées les uns avec les autres et si nécessaire, une mise à l'arrêt du dispositif est initiée.

15. Procédé selon l'une quelconque des revendications 9 à 14, **caractérisé en ce que** des valeurs de température dans la zone de la partie cutanée, déterminées avant, le cas échéant pendant et après l'action de la force sont comparées les unes avec les autres et / ou **en ce que** des valeurs de sérotonine déterminées avant et après l'action de la force sont comparées les unes avec les autres.

16. Procédé selon l'une quelconque des revendications 9 à 15, **caractérisé en ce qu'**aux fins d'une évaluation généralisée, des collectes de données concernant des temps de cycles de traitement, des répétitions des actions de la force sur des parties cutanées analogues d'êtres vivants présentant des caractéristiques analogues et un tissu corporel pathologique analogue sont créées et utilisées pour établir des algorithmes.
